# EUROPÄISCHE PATENTSCHRIFT

(11) **EP 2 079 527 B1**
(45) Veröffentlichungstag und Bekanntmachung des Hinweises auf die Patenterteilung: **04.12.2013**
(21) Anmeldenummer: 07817690.6
(22) Anmeldetag: 18.10.2007
(51) Int. Cl.: A61P 35/00, A61K 33/00, A61K 9/00

(54) **Verwendung von Deuteriumdioxid zur Behandlung von nicht-malignen hyperproliferativen Erkrankungen der Haut**
Use of deuterium dioxide for treating non-malignant hyperproliferative skin diseases
Utilisation de dioxyde de deutérium pour traiter des maladies non-malignes hyperprolifératives de la peau

(30) Priorität: 18.10.2006 DE 102006049185
(43) Veröffentlichungstag der Anmeldung: 22.07.2009
(73) Patentinhaber: D2 Bioscience Group Ltd, Hamilton, HM12 (BM)
(72) Erfinder: Bayerl, Prof. Thomas Maria, London SW73LR (GB)
(74) Vertreter: Ettmayr, Andreas
(86) Internationale Anmeldenummer: PCT/DE2007/001855
(87) Internationale Veröffentlichungsnummer: WO 2008/046407

(56) Entgegenhaltungen:
- WO-A-96/03996
- WO-A-99/62510
- WO-A-2005/016234
- WO-A-2006/022460
- US-A1- 2002 183 380
- US-A1- 2004 234 450
- ARANYI P: "DEUTERIUM ISOTOPE EFFECTS ON THE RATES OF STEROID GLUCO CORTICOID RECEPTOR INTERACTIONS", EUROPEAN JOURNAL OF BIOCHEMISTRY, vol. 138, no. 1, 1984, pages 89-92, ISSN: 0014-2956

## Beschreibung

Die vorliegende Erfindung betrifft die Verwendung von Deuteriumdioxid (D₂O) zur Prophylaxe und/oder Therapie von nicht-malignen hyperproliferativen Erkrankungen der Haut. Weiterhin betrifft die Erfindung D₂O-enthaltende Pflaster, Bandagen, Aerosole und Formulierungen zur gezielten topischen Applikation von D₂O auf der Haut.

Hyperproliferative Erkrankungen im Gewebe von Säugetieren zeichnen sich allgemein durch ein im Vergleich zu normal proliferierenden Zellen überdurchschnittliches Zellwachstum mit einer damit verbundenen erhöhten Zellteilungsrate aus. Von hyperproliferativen Erkrankungen können nahezu alle Gewebe- und Zelltypen betroffen sein, wie Leber-, Lungen-, Retina-, Darm-, Bauchspeicheldrüsen- und Hautgewebe bzw. -zellen. Bei den hyperproliferativen Erkrankungen der Haut wird zwischen malignen Erkrankungen, hierzu zählen insbesondere alle Krebserkrankungen, wie z. B. Melanom, Basalzellkarzinom, Platteneptithelkarzinom, kleinzellige Karzinome, Adenokarcinome, großzellige Karzinome, adenosquamöse Karzinome, sarkomatoide Karzinome, mucoepidermoide Karzinome, adenoid-zystische Karzinome und epithelial-myoepitheliale Karzinome und nicht-malignen Erkrankungen, wie z. B. insbesondere Psoriasis (Schuppenflechte), z.B. Psoriasis vulgaris, Psoriasis Pustulosa und Psoriasis-Arthritis, Keratosen, z.B. benigne lichenoide Keratose, palmoplantare Keratose, follikuläre Keratose, Verruca seborrhoica und Lichen-planus-artige Keratose, Akne, z.B. Akne vulgaris und Akne inversa, Porokeratosen, z.B. Porokeratosis disseminata, Porokeratosis Mibelli, Porokeratosis naeviformis, Porokeratosis striata und Porokeratosis disseminata, diabetisches Fußsyndrom und weiteren keratonisierenden Hautkrankheiten unterschieden. Weiterhin sind auch Narben der Haut in einigen Fällen, beispielsweise hypertrophe Narben und Keloide, die Folge einer Hyperproliferation des Bindegewebes der Haut, welche sich insbesondere durch vermehrte Bildung von Fibroblasten, dermalen Kollagen und Keratinozyten manifestiert. Allgemein wird als Narbe (lat. *cicatrix*) ein minderwertiges, faserreiches Ersatzgewebe bezeichnet, das den Endzustand einer Wundheilung darstellt.

Eine hypertrophe Narbe entsteht kurz nach der Wundheilung oder noch in deren Verlauf und ist eine, auf das Gebiet der ursprünglichen Wunde beschränkt bleibende, über das Hautniveau ragende, oft wulstige, erythematöse Verdickung der Haut mit spontaner, oft aber nicht kompletter, Rückbildung. Die Ursache ist eine Hyperproliferation von Bindegewebszellen. Hypertrophe Narben treten vermehrt dann auf, wenn eine Wunde nicht ruhiggestellt bzw. geschont wird oder wenn eine zusätzliche Infektion auftritt. Von der hypertrophen Narbe wird das Keloid unterschieden. Ein Keloid ist eine überschießende Narbenbildung auch außerhalb der ursprünglichen Wunde und stellt einen durch überschießendes Wachstum von Fibroblasten basierenden, die Haut überragenden gutartigen Tumor dar, der insbesondere nach Verletzungen, Operationen oder auch spontan auftreten kann, und ist als ein gestörter Heilungsprozess anzusehen. Keloide neigen nur in geringem Maß zur Rückbildung und entstehen auf genetischer Basis. Hypertrophe Narben und Keloide werden in der vorliegenden Erfindung zu den nicht-malignen hypertrophen Erkrankungen der Haut gezählt.

Eine weitverbreitete nicht-maligne hyperproliferative Erkrankung der Haut stellt Psoriasis dar. Allein in Deutschland sind ca. 2% der Bevölkerung, dies entspricht ungefähr 1,5 Mio. Menschen, von Psoriasis betroffen. Hierbei handelt es sich um eine chronische, nicht ansteckende Erkrankung der Haut. Während die Anlage für diese Erkrankung vererbt wird, sind die Auslöser jedoch äußerlich, beispielsweise Streß. Bei den verschiedenen Formen der Psoriasis, unterteilt in leichte, mittelschwere und schwere Psoriasis, handelt es sich in ca. 90 % aller Fälle um Psoriasis vulgaris, seltenere Fälle sind Psoriasis pustulosa und Psoriasis-Arthritis. Die komplexen bei der Psoriasis auftretenden Hautveränderungen sind durch 3 Faktoren gekennzeichnet:
a) typische epidermale Veränderungen,
b) typische Veränderungen des kutanen Gefäßsystems und
c) einem charakteristisch entzündlichen Infiltrat.

Bei Psoriasis sind insbesondere Keratinozyten hyperproliferativ mit veränderter Differenzierung, was durch Parahyperkeratose, aberrante Expression von Keratin 6/16, Involucrin, Filaggrin, sowie Integrin-Adhäsionsmolekülen (VLA-3, VLA-5, VLA-6, a6b4) charakterisiert ist. Außerdem exprimieren die Keratinozyten de-novo MHC Klasse II- und ICAM-1-Moleküle. Dies sind Moleküle, welche die Interaktion mit Leukozyten vermitteln. Die Endothelaktivierung in psoriatischer Haut führt zu Vasodilatation, Angiogenese sowie gesteigerter Expression von MHC Klasse II-, ICAM-1-, E-Selektin-, VCAM-1- und anderen Rezeptoren. Schließlich findet sich in psoriatischen Läsionen ein Infiltrat aus aktivierten Lymphozyten in Dermis und Epidermis, Neutrophilen in der Dermis und in epidermalen Munro-Mikroabszessen, sowie Mastzellen und Makrophagen. Vor allem T-Lymphozyten spielen eine primäre Rolle bei der Pathogenese psoriatischer Hautveränderungen. Diese Ansicht wird gestützt durch die Assoziation von Psoriasis mit bestimmten MHC-Allelen, den therapeutischen Effekt T-Zell-suppressiver Substanzen, wie Cyclosporin A oder dem lymphozytenspezifischen Toxin DAB389IL-2, die Verbindung eines bei Psoriasis-Patienten gesteigert exprimierten Gens mit einem Interleukin IL-2-regulierenden Gen, das Abheilen psoriatischer Läsionen nach Knochenmark-Transplantationen und das Ansprechen auf eine Therapie mit CD4-spezifischen Antikörpern. In einigen Fällen wurde eine oligoklonale Expansion als Hinweis auf antigenspezifische Reaktionen nachgewiesen, jedoch konnte bislang kein Autoantigen sicher identifiziert werden.

Auch neutrophile Granulozyten spielen eine wichtige Rolle bei der Pathogenese von Psoriasis. Es wird angenommen, dass Neutrophile durch das Zusammenwirken chemotaktischer Faktoren zur Migration in psoriatische Läsionen stimuliert werden. Durch GM-CSF, das in psoriatischen Läsionen verstärkt exprimiert wird, kann das Integrin aMb2 auf Neutrophilen induziert werden. Diese binden dann an Liganden (beispielsweise ICAM-1) auf Endothel- und aktivierten Epidermiszellen. Die Beobachtung, dass Munro-Mikroabszesse fast ausschließlich in parakeratotischen Arealen lokalisiert sind, führte zu der Vermutung, dass Neutrophile die Keratinozyten-Differenzierung beeinflussen können. Die stimulierten (aktivierten) neutrophilen Granulozyten produzieren reaktive Sauerstoff-Radikale und Proteasen, welche die Keratinozyten-Proliferation beeinflussen, Antigene demaskieren, Komplement aktivieren oder Gewebebestandteile degradieren könnten. Schließlich können Neutrophile durch Sekretion verschiedener Lipid-Mediatoren die DNS-Synthese in Keratinozyten stimulieren. Im Mausmodell konnte eine pathogenetische Punktion neutrophiler Granulozyten bei psoriatischen Hautveränderungen bestätigt werden. Die psoriatischen Gewebeveränderungen werden durch ein Netzwerk verschiedener Zytokine vermittelt. Hinzu kommen zahlreiche Chemokine, welche besonders für die gewebespezifische Lokalisation von Leukozyten eine wesentliche Rolle spielen.

Die Rolle der durch Adhäsionsrezeptoren vermittelten gewebespezifischen Leukozytenlokalisation bei Psoriasis ist dagegen bisher nur in Ansätzen bekannt und basiert auf dem Zusammenspiel verschiedener molekularer Interaktionen. Bei der Extravasation gehen Leukozyten zunächst transiente Bindungen mit Endothelien ein, die durch Selektine vermittelt werden. "Rollende" Leukozyten können durch b2-Integrine fest an Liganden aus der Immunglobulin-Superfamilie binden. Auch bl-Integrine und ihre Liganden sind an fester Leukozyten-Endothel-Bindung beteiligt. Haben Leukozyten das "Gefäßbett" verlassen, bewirken vor allem bl-Integrine die Bindung an ECM-Proteine.

Diese zahlreichen Mosaiksteine der Pathogenese der Psoriasis erlauben jedoch kein klares Gesamtbild dieser komplexen Hautkrankheit. Entsprechend schwierig ist ihre Prophylaxe und/oder Therapie, welche bis heute keine befriedigenden Ergebnisse vorweist, und in den meisten Fällen bleibt Psoriasis langfristig nicht therapierbar. Die Therapie von Psoriasis wird insbesondere durch zwei wesentliche Faktoren erschwert:
- zum einen handelt es sich um eine chronisch rezidivierende Erkrankung, die über einen sehr langen Zeitraum eine Behandlung erfordern kann,
- zum anderen müssen Individualfaktoren wie internistische Begleiterkrankungen (Diabetes, Hypertonus, Leberschäden), ebenso wie die klinischen Formen der Psoriasis und ihre Akuität, der Hauttyp (Pigmentation) und Vorbehandlung beachtet werden.

Die Therapie von Psorisasis erfolgt allgemein als Lokaltherapie oder/und systemische Therapie sowie in Form einer Phototherapie, die vorzugsweise mit anderen Therapieformen kombiniert werden kann (Lehmann P, Ruzicka T: Neue Entwicklungen in der Psoriasistherapie. Dt ärztebl 1996; 93: A-3188-3193). US 2004 234450 offenbart die Verwendung von D₂O als Excipient gegen Keratose, Warren und Krebs.

Bei der lokalen antipsoriatischen Therapie ist Anthralin (Dithranol, Cignolin) vor allem bei stationär und in Tageskliniken behandelten Patienten auch heute noch ein viel verwendetes Arzneimittel. Es wird zunächst in niedrigen Konzentrationen auf die Haut aufgebracht und die Konzentration wird dann schrittweise erhöht. Die Therapie kann als Langzeittherapie oder Kurzkontakt-Behandlung durchgeführt werden. Einschränkungen bezüglich der Therapiedauer oder der befallenen Körperoberfläche bestehen für Anthralin nicht. Lokale Irritationen (Cignolin-Dermatitis) können anfangs oder bei zu schneller Steigerung der Anthralin-Konzentration auftreten. Nachteilig ist die bei einer Anthralin-Therapie, vor allem bei höheren Konzentrationen auftretende, Verfärbung von umgebender Haut (sogenanntes Anthralin-Braun, ein Oxidationsprodukt des Wirkstoffs). Die topische Therapie der Psoriasis mit Analoga des Vitamin D3 hat in den letzten Jahren einen Stellenwert vor allem in der ambulanten Behandlung erhalten (van de Kerkhof PC: An update on vitamin D3 analogues in the treatment of psoriasis. Skin Pharmacol Appl Skin Physiol 1998; 11: 2-10.). Die in verschiedenen galenischen Formen einsetzbaren Vitamin-D3-Analoga Calcipotriol und Tacalcitol werden vor allem bei der chronisch-stationären Psoriasis (Plaque-Typ) eingesetzt. Bedingt durch die gute antiproliferative und differenzierungsinduzierende Wirkung kommt es zu einer Verminderung der Schuppung und Infiltration. Vitamin-D-Präparate eignen sich daher gut für die Kombination mit einer UV-B-Photatherapie. Das topische Retinoid Tazarotene ist neu in der Psoriasistherapie, Erfahrungen mit diesem Wirkstoff sind daher noch begrenzt. Topische Kortikosteroide sind die am häufigsten verwendeten Arzneimittel zur Therapie der Psoriasis. Vor allem bei den entzündlichen Formen haben sich Kortikosteroide bewährt. Topische Kortikosteroide wirken jedoch nur kurzfristig, so daß mit einem Wiederauftreten von Psoriasisherden nach Absetzen zu rechnen ist.

Die systemische Therapie ist immer dann notwendig, wenn die Psoriasis große Teile des Integumentes einnimmt und die Krankheitsaktivität hoch ist (häufige Rezidive). Hierbei ergab Ciclosporin in zahlreichen Studien sehr gute Wirksamkeit bei schwerer Psoriasis vulgaris (Mrowietz U, Färber L, Henneicke von Zepelin HH, Bachmann H, Welzel D, Christophers E: Long-term maintenance therapy with ciclosporine and posttreatment survey in severe psoriasis: results of a multicenter study. German Multicenter Study. J Am Acad Dermatol 1995; 33: 470-475.). Auch pustulöse Psoriasis-Formen (Psoriasis pustulosa) und Psoriasis-Arthritis können erfolgreich mit Ciclosporin behandelt werden. Ciclosporin hemmt die Aktivität von T-Zellen, Antigen-präsentierenden Zellen und Mastzellen und beeinflußt somit wesentliche Effektorzellen der psoriatischen Gewebereaktion. Als unerwünschte Nebenwirkung steht jedoch eine dosisabhängige Einschränkung der Nierenfunktion und die Entwicklung eines Hypertonus im Vordergrund.

Für eine weitere systemische Therapie von Psoriasis kommt die Behandlung mit aromatischen Retinoiden in Frage. Hier ist u.a. der Wirkstoff Acitretin zugelassen, der das vorher im Handel befindliche Etretinat abgelöst hat. Eine Monotherapie mit Acitretin ist zwar bei der Plaque-Typ-Psoriasis nicht so wirksam wie andere systemische Arzneimittel, jedoch können bei pustulösen Psoriasis-Formen (Psoriasis pustulosa) gute Behandlungsresultate erzielt werden. Eine verbesserte Wirkung ist durch die (etablierte) Kombination mit PUVA ("Re-PUVA") möglich. Als unerwünschte Nebenwirkungen können Hauttrockenheit, diffuse Alopezie, Knochen- und Muskelschmerzen auftreten. Laborchemisch kann eine Erhöhung der Serumlipide und/oder ein Anstieg der Leberenzyme auftreten.

Weiterhin werden Fumarsäureester seit Jahren zur Therapie der schweren Psoriasis verwendet. Die heute zur Therapie zugelassenen Präparate Fumaderm initial und Fumaderm enthalten mehrere Ester der Fumarsäure in unterschiedlicher Konzentration (Christophers E, Mrowietz U: Psoriasis. In: Freedberg IM, Eisen AZ, Wolff K, Austen KF, Goldsmith LA, Katz S1, Fitzpatrick TB, Hrsg.: Dermatology in General Medicine. New York: McGraw-Hill 1999; 495-521.). Starke unerwünschte Nebenwirkungen sind jedoch vor allem Magen-Darm-Beschwerden und das Auftreten einer Flush-Symptomatik. Bei längerer Therapie können darüber hinaus ein Absinken der Leukozytenzahlen mit Lymphopenie und eine Eosinophilie beobachtet werden.

Bei den Phototherapien wirken sowohl UV-B- als auch UV-A-Licht lokal immunsuppressiv und führen zu zahlreichen Effekten, insbesondere in der Epidermis und Dermis. Dabei unterscheiden sich UV-A- und UV-B-Licht besonders durch die zur Erzielung einer Reaktion erforderliche "minimale Erythem-Dosis", (MED). Die Kombination von UV A mit einem Photosensibilisator (PUVA-Therapie) weist als Photochemotherapie den stärksten antipsoziatischen Effekt auf. Die PUVA-Therapie kann als orale oder als Bade-PUVA-Therapie durchgeführt werden. Bei der klassischen (oralen) PUVA-Behandlung wird der Photosensibilisator (meist 8-Methoxypsoralen) oral eingenommen. Nach Resorption des Wirkstoffs und Anreicherung in der Haut innerhalb von zwei Stunden erfolgt die UV-A Bestrahlung. In den letzten Jahren hat sich besonders die Bade-PUVA-Therapie bewährt, bei der der Photosensibilisator über das Badewasser der Haut zugeführt wird. Die Vorteile liegen darin, dass keine systemische Wirkung des Photosensibilisators erfolgt und eine insgesamt geringere Gesamt-UV-A-Dosis erforderlich ist. Auch die Anwendung des Photosensibilisators in einer geeigneten Cremegrundlage zur gezielten Therapie umschriebener Psoriasisherde ("Creme-PUVA") ist möglich. Weiterhin hat eine Kombination von Salzbädern (über fünf Prozent) mit nachfolgender UV-Bestrahlung (Sole-Phototherapie) in jüngster Zeit weitgehende Anwendung gefunden.

Insbesondere können darüber hinaus Kombinationen der vorstehend aufgeführten Therapieformen sehr wirkungsvoll in der Behandlung von Psoriasis sein (Kombinationstherapien). Die Kombination von Externa (d.h. extern applizierte Substanzen, wie Salben, Cremes und flüssige Wirkstoffe) mit UV-Therapie oder systemischen Arzneimitteln, z.B. Ciclosporin, Acitretin, Fumarsäureestern und Methotrexat (MTX), führt beispielsweise in der Regel zu einer wesentlich verbesserten Ansprechbarkeit der psoriatischen Effloreszenzen und kann auch zu einer Verlängerung der erscheinungsfreien Zeit führen. Bekannte, klassische Kombinationen hierzu sind die Anwendung von Stark-Solebädern mit anschließender UV-B-Bestrahlung (Sole-Phototherapie), die topische Therapie mit Kortikosteroiden oder Vitamin-D3-Analoga in Verbindung mit UV-B-Licht oder PUVA und die Kombination von systemischen Retinoiden mit PUVA (Re-PUVA). Zu beachten ist jedoch auch, daß zahlreiche systemische Arzneimittel, wie Ciclosporin, Acitretin, MTX (Methotrexat) und Fumarsäureester, aufgrund starker Nebenwirkungen nicht mit einer gleichzeitigen UV-Therapie kombiniert werden können.

Eine bedeutende Gruppe der nicht-malignen hyperproliferativen Erkrankungen der Haut stellen die Keratosen dar, die in der Regel mit einer beschleunigten Teilung von Hautzellen, insbesondere der Keratinozyten - einer Hyperkeratose - einhergehen. Die Hyperkeratose ist allerdings nicht in allen Erkrankungen, die vom Begriff "Keratose" umfasst sind, für die jeweiligen Erkrankungen kausal, fördert jedoch die Entstehung dieser Erkrankungen und beeinflusst die Entwicklung und die Schwere der Erkrankungen. In diesem Sinne unfasst der Begriff "Keratose" neben aktinischer Keratose, epidermolytischer Hyperkeratose, Hyperkeratosis Lenticularis Perstans, Keratosis pilaris Ichthyosen, benigner lichenoider Keratose, palmoplantarer Keratose, follikulärer Keratose, Verruca seborrhoica, Lichen-planus-artige Keratose und Porokeratose, insbesondere Porokeratosis disseminata, Porokeratosis mibelli, Porokeratosis naeviformis, Porokeratosis striata, Porokeratosis disseminata, auch Erkrankungen wie Akne, insbesondere Akne vulgaris, Akne inversa (je nach schwere der Ausprägung auch Akne comedonica, Akne papula-pustulosa und Akne conglobata genannt), Hidradentis suppurativea, Akne aestivalis, Akne cosmetica, Akne medicamentosa, Akne venenata und Akne tarda, die mit eine Hyperkeratose der terminalen Folikel bzw. eine follikulären Hyperkeratose apokriner Schweißdrüsen in Verbindung stehen (siehe z.B. Marks R. und Plewig G. (1988) Proceedings of an International Symposium on Akne and related disorders, Cardiff). Des Weiteren steht eine Reihe von Komplikationen, die bei Diabetes mellitus auftreten, mit Keratosen in Verbindung, so dass der Begriff Keratose unter anderem auch das diabetische Fußsyndrom umfasst.

Eine weitere nicht-maligne hyperproliferative Erkrankung der Haut von großer Bedeutung stellen Narben der Haut dar. Die im Stand der Technik bekannten Therapien von Narben der Haut, insbesondere hyperproliferativen Narben und Keloiden, können in folgende Therapiekonzepte unterteilt werden:
a) Injektion von Kortikoiden,
b) chirurgische Behandlung (Exzision),
c) Kompressionstherapie,
d) Strahlentherapie,
e) Lasertherapie,
f) Kryotherapie,
g) Aufbringung von hypoallergenen mikroporösen Pflastern ("Tapestreifen") mittels geeigneter Kleber und
h) Aufbringung von Silikongel-Folien.

Eine detaillierte Übersicht über vorgenannte Therapieformen und ihre durch Studien belegte Wirksamkeit wird beispielsweise in der Arbeit von Ziegler et al. (Ziegler et al., "International clinical recommendations on scar management." Plastic and Reconstructive Surgery, 2002, Bd. 110, No. 2, 560-571) gegeben. Danach konnte für jede dieser Therapien und auch für verschiedene Kombinationen davon eine Wirksamkeit nachgewiesen werden, in den meisten Fällen jedoch auch hier nur über einen begrenzten Zeitraum, nachdem zunächst eine (teilweise) Rezidivierung der behandelten Narben der Haut beobachtet wurde. Außerdem wurde nur in wenigen Fällen von einem vollständigen Verschwinden der Narben der Haut berichtet Der Verwendung von Silikonfolien wird ein besonders großes Potential zur präventiven Vermeidung bzw. Prophylaxe und/oder Therapie von Narben der Haut auf der Basis von randomisierten Kontrollstudien zugemessen. Auch Kombinationen der oben genannten Therapien mit einem Wirkstoff, welcher die Hyperproliferation hemmen oder begrenzen kann, erscheint als eine besonders wirksame Therapiemöglichkeit. Der Wirkstoff sollte jedoch auch hier möglichst nur lokal wirken und seine Nebenwirkungen auf das System des behandelten Organismus und auf die Wundheilung sollten vernachlässigbar sein.

Keine der im Stand der Technik beschriebenen Therapien von Narben der Haut greift jedoch in ihrem Wirkmechanismus an einer der wesentlichen Grundlagen der Narbenbildung an: Der Hyperproliferation von Dermis und/oder von Keratinozyten bzw. Fibroblasten im Narbenbereich.

Darüber hinaus ist ein allgemeines Problem aller im Stand der Technik aufgeführten Therapien von hyperproliferativen Erkrankungen der Haut ihre mangelnde Langzeitwirkung und ihre starken Nebenwirkungen. In der Regel führen sämtliche bekannten Therapien lediglich zu einer zeitlich begrenzten, kurzen symptomfreien Periode der Krankheit. Die dadurch erforderlichen zyklischen Wiederholungen der Therapie oder Therapiekombinationen führen zu einer Dauerbelastung des Systems mit in allen Fällen starken Nebenwirkungen. Darüber hinaus rufen nahezu alle systemisch verabreichten Arzneimittel, beispielsweise Kortikoide oder Zytostatika, auch schon bei einer kurzen Behandlungsdauer starke Nebenwirkungen hervor, die den gesamten Organismus des behandelten Patienten betreffen. Diese Nachteile des Standes der Technik betreffen nicht nur die vorstehend insbesondere beschriebenen hyperproliferativen Erkrankungen der Haut, nämlich Psoriasis sowie Narben der Haut, speziell hypertrophe Narben und Keloide, sondern erstrecken sich auf sämtliche maligne und nicht-maligne hyperproliferative Erkrankungen der Haut. Es besteht damit ein dringender Bedarf an neuen, verbesserten Wirkstoffen, deren Applikation zu keinen oder nur geringen bzw. vernachlässigbaren Nebenwirkungen führt und damit beliebig oft wiederholbar ist.

Aufgabe der vorliegenden Erfindung ist es demnach, verbesserte Wirkstoffe zur Behandlung nicht-malignen hyperproliferativer Erkrankungen der Haut bereitzustellen. Darüber hinaus ist es eine Aufgabe der vorliegenden Erfindung, verbesserte topische Applikationstechniken bereitzustellen.

Diese Aufgaben werden durch die vorliegende Erfindung gelöst. Die Erfindung betrifft in ihren ersten beiden Gegenständen die Verwendung von Deuteriumdioxid (D₂O) zur Prophylaxe und/oder Therapie von nicht-malignen hyperproliferativen Erkrankungen der Haut und die Verwendung von D₂O zur Herstellung eines Arzneimittels zur Prophylaxe und/oder Therapie von nicht-malignen hyperproliferativen Erkrankungen der Haut, Keratosen, wie z.B. aktinische Keratose, epidermolytische Hyperkeratose, Hyperkeratosis Lenticularis Perstans, Keratosis pilaris Ichthyosen, benigne lichenoide Keratose, palmoplantare Keratose, follikuläre Keratose, Verruca seborrhoica, Lichen-planus-artige Keratose und Porokeratosen, z.B. Porokeratosis disseminata, Porokeratosis Mibelli, Porokeratosis naeviformis, Porokeratosis striata, Porokeratosis disseminata, Akne, z.B. Akne vulgaris, Akne inversa (je nach schwere der Ausprägung auch Akne comedonica, Akne papula-pustulosa und Akne conglobata genannt), Hidradentis suppurativea, Akne aestivalis, Akne cosmetica, Akne medicamentosa, Akne venenata und Akne tarda, Hyperkeratosen im Zusammenhang mit Diabetes mellitus, wie z.B. diabetisches Fußsyndrom; , sowie Narben der Haut, insbesondere hypertrophe Narben und Keloide.

Die Begriffe "Prophylaxe" und/oder "Therapie" bezeichnen jede für die Behandlung einer nicht-malignen hyperproliferativen Erkrankung der Haut geeignete Maßnahme, die entweder eine vorbeugende Behandlung (Prophylaxe) einer solchen sich abzeichnenden Erkrankung bzw. deren sich abzeichnende Symptome oder zur Vermeidung eines Wiederausbrechens einer solchen Erkrankung, beispielsweise nach einem abgeschlossenen therapeutischen Behandlungszeitraum, betrifft oder der Behandlung der Symptome einer solchen bereits ausgebrochenen Erkrankung betrifft (Theraphie).

"Hyperproliferative Zellen" im Sinne der vorliegenden Erfindung bezeichnen Zellen, die die Fähigkeit zu einem autonomen, anormalen Wachstum, d.h., einem rapide proliferierenden Wachstum, besitzen. Hierbei sind pathologische Zustände, d.h. Zustände, die eine Erkrankung darstellen, und nicht-pathologische Zustände, d.h. Zustände, die eine Abweichung von der normalen Zellproliferationsrate darstellen, aber nicht mit einer Erkrankung des Gewebes assoziiert sind, z.B. Zunahme der Zellteilungsrate bei Wundheilung zu verstehen. Die hierin beschriebenen hyperproliferativen Zellen von Narben der Haug, insbesondere hypertrophen Narben und Keloiden, und deren Wachstum werden/wird den pathogenen Zuständen zugeordnet.

"Hyperproliferative Erkrankungen" oder "neoplastische Erkrankungen" der Haut bezeichnen im Sinne der Erfindung maligne und nicht-maligne Erkrankungszustände.

"Maligne" Erkrankungszustände schließen u.a. alle Arten tumor- oder krebsartigen Zellwachstums, onkogene Prozesse und maligne transformierte Zellen, Gewebe oder Organe ein, z. B. Karzinom, Sarkom oder Metastasen. Beispiele sind Melanom, Basalzellkarzinom, Platteneptithelkarzinom, kleinzellige Karzinome, Adenokarzinome, großzellige Karzinome, adenosquamöse Karzinome, sarkomatoide Karzinome, mucoepidermoide Karzinome, adenoid-zystische Karzinome und epithelial-myoepitheliale Karzinome.

Zu den "nicht-maligen" Erkrankungen gehören insbesondere Psoriasis, z.B. Psoriasis vulgaris, Psoriasis guttata, Psoriasis inversa, Psoriasis capitis, Psoriasis pustulosa und Psoriasis-Arthritis; Keratosen, z.B. benigne lichenoide Keratose, palmoplantare Keratose, follikuläre Keratose, Verruca seborrhoica und Lichen-planus-artige Keratose, Porokeratosen, z.B. Porokeratosis disseminata, Porokeratosis Mibelli, Porokeratosis naeviformis, Porokeratosis striata und Porokeratosis disseminata, Akne, z.B. Akne vulgaris, Akne inversa (je nach schwere der Ausprägung auch Akne comedonica, Akne papula-pustulosa und Akne conglobata genannt), Hidradentis suppurativea, Akne aestivalis, Akne cosmetica, Akne medicamentosa, Akne venenata und Akne tarda, und Hyperkeratosen im Zusammenhang mit Diabetes mellitus, wie z.B. das diabetisches Fußsyndrom; sowie Narben der Haut, insbesondere hypertrophe Narben und Keloide.

Als Voraussetzung für eine effektive Prophylaxe und/oder Therapie von hyperproliferativen Erkrankungen der Haut sollte ein pharmazeutischer Wirkstoff - unter Berücksichtigung der Art seiner Applikation - allgemein folgende Anforderungen erfüllen:
a) lokale Anwendbarkeit durch topische Applikation über einen beliebig langen Zeitraum (für topisch zu applizierende pharmazeutische Wirkstoffe);
b) weitgehend homogene Wirkstoffverteilung im Bereich des Wirkortes sowie die Vermeidung von lokalen Überkonzentrationen;
c) bevorzugte Anreicherung des pharmazeutischen Wirkstoffes in den hyperproliferativen Hautgebieten, verbunden mit einer Retardierung seines transdermalen Transportes ins System, d.h. in die Blutgefäße;
d) bevorzugte Wirkung auf hyperproliferative Zellen im betroffenen Hautgebiet im Sinne einer überproportionalen Hemmung, vorzugsweise Inhibierung, ihrer Wachstumsrate im Vergleich zu umliegenden nicht-hyperproliferativen Zellen und
e) weitgehende Toleranz der gesunden Zellen im Hautgewebe wie auch im gesamten System gegenüber dem pharmazeutischen Wirkstoff zur Vermeidung von Nebenwirkungen, insbesondere auch im Hinblick auf Immunreaktionen.

Die erfindungsgemäße Verwendung von D₂O als pharmazeutischen Wirkstoff - allein oder in Kombination mit mindestens einem weiteren pharmazeutischen und/oder mindestens einem weiteren nicht-pharmazeutischen Wirkstoff - sowie dessen gerichtete topische Applikation auf ausgewählten Bereichen der Haut, erfüllt alle diese Anforderungen und hat darüber hinaus gegenüber den im obigen Stand der Technik bekannten Therapien und pharmazeutischen Wirkstoffen einen deutlichen Vorteil hinsichtlich seiner topischen Applikation, der gerichteten lokalen Wirksamkeit und damit verbunden der Vermeidung einer Belastung des Systems (d.h. des Blutkreislaufs) des behandelten Organismus. Hierauf wird nachfolgend näher eingegangen.

Die vorliegende Erfindung basiert demnach auf der Erkenntnis, dass Deuteriumdioxid, nachfolgend mit D₂O bezeichnet, ein effektiver Wirkstoff mit potentieller Langzeitwirkung und geeignet für eine Langzeit-Therapie von hyperproliferativen Hauterkrankungen ist, der die im Stand der Technik bekannten starken Nebenwirkungen verringert, vorzugsweise ausschaltet. Wie bereits erwähnt, weisen hyperproliferative Zellen eine weitaus höhere Zellteilungsrate auf als normal proliferierende Zelten. Da Zellen bei ihrer Teilung Wasser, nachfolgend mit H₂O bezeichnet, aufnehmen, nehmen hyperproliferative Zellen aufgrund ihrer erhöhten Zellteilungsrate deutlich mehr H₂O auf als normal proliferierende Zellen. D₂O, häufig auch als "schweres Wasser" bezeichnet, ist eine dem "natürlichen Wasser" H₂O äußerst ähnliche Substanz und wird von sich teilenden Zellen anstelle (wenn nur D₂O verfügbar ist) oder parallel zu H₂O (wenn D₂O und H₂O verfügbar sind) aufgenommen. Die Gründe hierfür werden nachfolgend näher erläutert.

D₂O und H₂O unterscheiden sich physikalisch durch die Substitution der Wasserstoffatome von H₂O durch Deuteriumatome, wobei D₂O eine ca. 10% höhere Dichte und eine ca. 25% höhere Viskosität aufweist. Darüber hinaus sind Schmelz- und Siedetemperaturen von D₂O höher als für H₂O. Ein detaillierter Vergleich der Eigenschaften wird im Handbook of Chemistry and Physics, Sektion 6, gegeben (Handbook of Chemsitry and Physics, David R. Lide, Editor, 79. Auflage, 1998 CRC Press, Boca Raton, USA) dargestellt.

Während die physikalischen Unterschiede zwischen H₂O und D₂O eher gering sind, bestehen bedeutsame physiologische Unterschiede (siehe u.a. Kushner DJ et al., Pharmacological uses and perspectives of heavy water and denatured compounds., Can J Physiol Pharmacol. 1999 Feb;77(2):79-88.). So wurde beispielsweise festgestellt, dass, obwohl viele Algen und Bakterien in 100% D₂O völlig normal über lange Zeiten existieren können, und für Protozoen dies bei bis zu 70% D₂O möglich ist, dies nicht für tierische Zellen gilt. Hier werden bei D₂O-Konzentrationen im Organismus von mehr als 20 - 25% verschiedene enzymatisch gesteuerte Reaktionen zunehmend verändert, insbesondere inhibiert. Eine Ursache hierfür ist vermutlich die höhere Bindungsstärke der Wasserstoffbrückenbindungen, wenn das Wasserstoffatom von H₂O durch ein Deuteriumatom substituiert ist. Sowohl in wässrigen Lösungen von H₂O und D₂O, als auch bei der Bindung von Wasser an organische Moleküle, tritt diese erhöhte Bindungsstärke auf, wobei bei organischen Molekülen der Effekt noch stärker ausgeprägt zu sein scheint (Cuma M, Scheiner S, Influence of Isotopic Substitution on Strength of Hydrogen Bonds of Common Organic Groups, Journal of Physical Organic Chemistry, 1997 Band 10, 383-395).

Ein wichtiger, auf dieser veränderten Bindungseigenschaft basierender Aspekt ist die Erkenntnis, dass erhöhte Konzentrationen von D₂O die Zellteilung hemmen oder gänzlich inhibieren können. Dies geschieht wahrscheinlich hauptsächlich durch die Inhibierung der DNA-Synthese (Takeda H et al. Mechanisms of cytotoxic effects of heavy water (deuterium oxide: D2O) on cancer cells, Anticancer Drugs. 1998 Sep;9(8):715-25) bzw. der Mitose im Zyklus der Teilung tierischer Zellen (Laissue JA et al. Survival of tumor-bearing mice exposed to heavy water or heavy water plus methotrexate, Cancer Research, 1982, Vol 42, (3) 1125-1129).

Wie vorstehend ausgeführt,
- hat D₂O bei geeigneter Konzentration in einer proliferierenden Zelle unter anderem die Fähigkeit, die Zellteilung zu hemmen bzw. inhibieren;
- nehmen proliferiernde Zellen D₂O ebenso wie H₂O auf; und
- nehmen hyperproliferative Zellen aufgrund ihrer erhöhten Zellteilungsrate überptoportional mehr D₂O als normal proliferierende Zellen auf.

Daraus ergibt sich, dass D₂O in einem tierischem Gewebe, vorzugsweise von einem Säugetier, in welchem selten oder normal proliferierende Zellen neben einem Bereich von hyperproliferierenden Zellen vorhanden sind, - anstelle oder zusätzlich zu H₂O - in den hyperproliferativen Zellen durch aktiven bzw. passiven Transport angereichert wird, wodurch deren Zellteilungsrate überproportional verlangsamt, gehemmt oder sogar gänzlich inhibiert wird. Dieser Effekt ist bei der Therapie zahlreicher medizinischer Indikationen hyperproliferativer maligner, wie z.B. Tumortherapie, und nicht-maligner Erkrankungen, nicht nur sehr erwünscht, sondern ein stark angestrebtes Ziel, da hyperproliferative Zellen ein unkontrolliertes und in den meisten Fällen schädliches Wachstum im Gewebe bewirken.

Für den Fall des Tumorwachstums im Dickdarm und in den Plattenepithelzellen im Mund- und Rachenraum wurde diese Wirkung von D₂O bereits in den 80-iger Jahren experimentell an Balb/c/-nu/n u Mäusen nachgewiesen (Alternatt HJ et al., Heavy water delays growth of human carcinoma in nude mice; Cancer. 1988 Aug 1;62(3):462-6.) Es wurden Versuchstiere mit Trinkwasser versorgt, welches mit 20-30% D₂O (bezogen auf das Gesamtvolumen des Trinkwassers) angereichert war. Die nachweisbare Antitumorwirkung durch D₂O im Trinkwasser erstreckte sich jedoch nur über den Zeitraum der Verabreichung dieses Wassergemisches aus D₂O und H₂O, nach Übergang zu normaler H₂O-Verabreichung war keine Nachhaltigkeit der Antitumorwirkung festzustellen.

Auch in anderen Geweben konnte eine Antitumorwirkung nachgewiesen werden. So unterstützen kürzlich publizierte Zellkultur-Studien mit 3 Tumorzellinien aus der Bauchspeicheldrüse (Pancreas) ebenfalls eine Antitumorwirkung (Hartmann, J. et al., Effects of heavy water (D2O) on human pancreatic tumor cells, Anticancer Res. 2005 Sep-Oct;25(5):3407-11). Andere Untersuchungen ergaben eindeutige Resultate für die D₂O-vermittelte Antitumorwirkung in Geweben von neoplastischen Hirnzellen und legen nahe, dass D₂O eine Apoptose in malignen Astrozytomzellen induzieren kann (Uemura, T. et al, Experimental validation of deuterium oxide-mediated antitumoral activity as it relates to apoptosis in murine malignant astrocytoma cells., Neurosurg. 2002 May;96(5):900-8.). Auch in dieser Arbeit schlussfolgerten die Autoren, dass D₂O zytotoxisch auf Tumorgewebe wirkt und dadurch ein Zytostatikum darstellt. Auch publizierte Studien, bei denen ein etabliertes Zytostatikum zusammen mit D₂O anstelle von H₂O zur Behandlung von murinen, xenotransplantierten Tumoren verabreicht wurde (Altermatt, HJ, Heavy water (D2O) inhibits growth of human xenotransplanted oropharyngeal cancers. An animal experiment study in nude mice, Laryngol Rhinol Otol (stuttg). 1987 Apr;66(4):191-4.), bestätigen eine zusätzliche anti-neoplastische Wirkung von D₂O.

Dennoch wurde bislang im Stand der Technik ein therapeutischer Nutzen der systemischen Verabreichung von D₂O an einen tierischen Organismus, insbesondere eines Säugetiers, zur Behandlung von hyperproliferativen Erkrankungen trotz der oben erwähnten vorteilhaften zytotoxischen Wirkung abgelehnt, da die für eine solche Wirkung erforderlichen D₂O-Konzentrationen über 25% liegen und damit starke Nebenwirkungen, beispielsweise einer Veränderung der Proteinsynthese oder der Proteinfaltung erwartet wurden (Kushner DJ et al., Pharmacological uses and perspectives of heavy water and denatured compounds., Can J Physiol Pharmacol. 1999 Feb;77(2):79-88.). Darüber hinaus kommen alle im Stand der Technik publizierten Studien zur D₂O-Wirkung an Zellen, Organen und Organismen zu der Schlussfolgerung, dass die Wirkung von D₂O nur für die Zeitdauer der D₂O-Verabreichung anhält, wodurch sehr lange Zyklen der D₂O-Verabreichung notwendig wären. Daraus ergäbe sich wiederum eine hohe Belastung des gesamten behandelten Organismus mit D₂O und damit verbundene Nebenwirkungen, wie vorstehend erwähnt. Dies ist ein weiterer Aspekt, weshalb bisher keine Therapien von hyperproliferativen Erkrankungen mit D₂O als Wirkstoff im Stand der Technik beschrieben sind.

Nirgendwo im Stand der Technik ist demnach bislang eine wirksame Prophylaxe und/oder Therapie von nicht-malignen hyperproliferativen Erkrankungen insgesamt, und in besonderem der Haut, durch systemische oder nicht-systemische -beispielsweise topische Applikation auf die Haut - Verabreichung von D₂O beschrieben.

Erfindungsgemäß ist es jedoch nun gelungen, die im Stand der Technik nach systemischer D₂O-Verabreichung beschriebene anti-neoplastische Wirkung bzw. Antitumorwirkung von D₂O auf eine wirksame topische D₂O-Applikation auf die Haut zur Behandlung hyperproliferativer Erkrankungen der Haut zu übertragen. Dies liegt insbesondere in folgenden einzigartigen Eigenschaften von D₂O als pharmazeutischen Wirkstoff begründet die es gegenüber allen anderen pharmazeutischen und insbesondere zytotoxischen Wirkstoffen auszeichnet:
1) durch die Möglichkeit, D₂O topisch auf die Haut zu applizieren, kann eine zur therapeutischen Wirksamkeit ausreichend hohe Konzentration von D₂O in der Epidermis bzw. Dermis der Haut erreicht werden, ohne dass andere Organe des Körpers ähnlich hohe (und für sie möglicherweise schädliche) Konzentrationen von D₂O erfahren müssen. Damit ist ein entscheidendes im Stand der Technik diskutiertes Problem der Erreichung von therapeutisch wirksamen D₂O-Konzentrationen am Wirkort (meist über 20% D₂O bezogen auf den Gesamtwassergehalt der Zelle) ohne starke Nebenwirkungen für andere Organe oder gesundes Hautgewebe gelöst. Grundlage hierfür ist der gerichtete Transport von D₂O durch das Stratum Corneum der Haut bis zur Epidermis bzw Dermis;
2) der Aggregatzustand von D₂O kann bei einer topischen Verabreichung flüssig, gasförmig oder fest sein, der Transport in die Haut kann durch direkten Kontakt der Wirkstoffzusammensetzung mit der Haut wie auch indirekt durch Diffusion über eine zwischengelagerte Schicht (z.B. Luft, poröse Membran, polymeres Netzwerk) erfolgen;
3) insbesondere für den Fall, dass reines flüssiges D₂O allein verabreicht wird (reines D₂O), ist auszuführen, dass D₂O gegenüber allen anderen flüssigen pharmazeutischen Wirkstoffen einen einzigartigen Vorteil hat. Es kann wie normales Wasser (H₂O) in die Haut transportiert werden und durch die Stärke und Richtung des osmotischen Gradienten und einer Manipulation dieser beiden Größen kann weiterhin die Eindringtiefe von D₂O in die Haut an die therapeutische Zielsetzung angepasst werden;
4) die Wasserstoffbrückenbindungsstärke von D₂O ist, wie bereits oben beschrieben, höher als von H₂O, insbesondere bei der Bindung von Wasser an organische Moleküle. Topisch verabreichtes D₂O bindet molekular durch Wasserstoffbrückenbindungen an die nächste verfügbare Zelloberfläche und verdrängt dabei das dort angelagerte H₂O aufgrund seiner höheren Bindungsstärke. Die Austausch-Frequenz der D₂O-Moleküle mit der H₂O-Umgebung ist wiederum durch diese erhöhte Bindungsstärke (und durch das höhere Gewicht des D₂O-Moleküls) etwas langsamer als für H₂O (König, S.,et al. "Molecular dynamics of water in oriented multilayers studied by quasi-elastic neutron scattering and deuterium-NMR relaxation".1999. J.Chem. Phys. 100, 3307-3316). Damit ergibt sich eine erhöhte Aufenthaltswahrscheinlichkeit der D₂O-Moleküle unmittelbar auf der Zelloberfläche. Als Konsequenz resultiert eine bevorzugte Internalisierung von D₂O in der Zelle, wodurch es; im Zytoplasma angekommen, seine auf Inhibierung der Zellteilung gerichtete Wirkung entfalten kann. Damit wird D₂O für den Fall der Haut tatsächlich im Bereich der Keratinozyten angereichert und nicht mit der Transportgeschwindigkeit von H₂O ins System entlassen. Dies ist ein für die Wirksamkeit entscheidender Schritt, da nur höhere Konzentrationen an D₂O in der Zelle tatsächlich eine Reduzierung der Zellteilung zur Folge haben. Da hyperproliferative Zellen (benigne und maligne) meist eine höhere Permeabilität bzw. Aufnahmefähigkeit für Wasser aufweisen als normale Zellen, ist gleichzeitig gesichert, dass D₂O überproportional in diesen Zellen im Vergleich zu normal proliferierenden Zellen angereichert wird und dort das Zellwachstum entsprechend reduziert oder inhibiert. Aufgrund dieser Eigenschaften ist die topische Applikation von D₂O auf der Haut außerordentlich wertvoll für die Therapie von hyperproliverativen Erkrankungen, bei denen der gewünschte Wirkort in der Epidermis oder höchstens in der Dermis der Haut liegt;
5) D₂O ist das einzige nicht-radioaktive Molekül, welches dem H₂O in seinen Eigenschaften sehr ähnlich ist. Zellen allgemein, und insbesondere Keratinozyten der Haut, können zwischen den beiden Molekülen nicht "unterscheiden", so dass D₂O durch aktiven und passiven Transport auf die gleiche Weise wie H₂O in die Zelle transportiert wird und bis in die Zellkerne gelangt. Hierdurch werden Zellbarrieren beliebiger Arten, die ein Eindringen anderer pharmazeutischer Wirkstoffe verhindern, umgangen und ebenfalls Abwehrmechanismen auf zellulärer Ebene, wie die Internalisierung in Lysosomen oder die Aktivierung von MDR (multiple drug resistance) Transportem oder auf Organebene durch das Immunsystem, welche die Wirksamkeit des pharmazeutischen Wirkstoffs D₂O reduzieren oder inhibieren könnten, sind weitgehend ausgeschaltet;
6) ein weiterer Vorteil von D₂O als pharmazeutischer anti-hyperproliferativer Wirkstoff ist die Tatsache, dass Konzentrationen unter 20% D₂O (bezogen auf den Gesamtwassergehalt der Zelle) in der Zelle keine signifikanten Wirkungen entfalten und damit normale Zellen, die wegen ihrer gegenüber den hyperproliferativen Zellen geringeren Wasserpermeabilität und/oder Wasseraufnahme vergleichsweise wenig D₂O aufnehmen, kaum den Wirkungen des D₂O ausgesetzt sind; und
7) hyperproliferative, nicht-maligne Hautkrankheiten, wie Psoriasis, zeichnen sich durch vermehrtes Zellwachstum, insbesondere der Keratinozyten, bei gleichzeitiger Infiltration von Lymphozyten aus. Da D₂O in der Lage ist, die Prolifieration von Zellen zu hemmen bzw. inhibieren, kann wegen des erhöhten Wasserbedarfs von hyperproliferativen Zellen, wie Keratinozyten, eine differentielle Wirkung auf deren Proliferation im Vergleich zum umliegenden normal proliferierenden Zellen aus nicht-psoriatischem Gewebe erzielt werden. Der gleiche Effekt kann im Falle der Behandlung von hypertrophen Narben und Keloiden erzielt werden, da auch bei der Narbenbildung eine Hyperproliferation von insbesondere Fibroblasten und dermalen Kollagenzellen als Ursache für ein Wachstum des Narbengewebes über das Hautniveau hinaus angesehen wird.

Mit der vorliegenden Erfindung ist es ebenfalls gelungen, durch die erfindungsgemäße topische Applikation lokal hohe, therapeutisch wirksame D₂O-Konzentrationen auf der Haut einzusetzen und gleichzeitig die Belastungen des Systems (d.h. des Blutkreislaufs) und die Nebenwirkungen auf nicht zu behandelndes, gesundes Hautgewebe sowie auf Gewebe von anderen (nicht zu therapierenden) Organen des behandelten Organismus, wie z.B. der Leber oder Niere, (die durch eine hohe Konzentration von D₂O von mehr als 20% D₂O, bezogen auf den Gesamtwassergehalt der Zelle, verursacht werden können), zu vermindern oder sogar vollständig zu vermeiden. Ein Transport von D₂O in das System kann mit Mitteln, die im Stand der Technik gut bekannt sind, verhindert oder eingeschränkt werden kann. Beispiele für diese Mittel sind u.a. die gezielte Manipulation des osmotischen Gradienten über die Haut (d.h. zwischen systemischen Teil und der Hautoberfläche) durch Verringerung des Wasserpotentials des topisch applizierten D₂O mittels Substanzen, die geeignet sind, dieses Wasserpotential zu verändern, insbesondere physiologisch verträgliche Salze, wie Natriumchlorid, wasserlösliche Polymere und andere nicht-pharmazeutische Stoffe. Ein zu behandelnder Organismus im Sinne der vorliegenden Erfindung bezeichnet vor allem einen tierischen Organismus, insbesondere eines Säugetieres, eines humanen oder nicht-humanen Säugetiers, wie beispielsweise Mensch, Ratte, Maus, Pferd, Schwein, Schaf und Ziege.

So ist erfindungsgemäß nach topischer Applikation von D₂O als Wirkstoff auf eine an Psoriasis erkrankte menschliche Haut eine heilende Wirkung ohne systemische oder andere starke Nebenwirkungen nachgewiesen worden. Weiterhin konnte bei Narben der Haut, insbesondere bei hypertrophen Narben und Keloiden, durch die topische Applikation von D₂O eine Verringerung der Hypertrophie einer Narbe gezeigt werden. Auch konnte durch die Applikation von D₂O in Hautkulturen eine Hemmung oder Inhibierung des Wachstums maligner und nicht-maligner hyporproliferativer Zellen nachgewiesen werden. Schließlich konnten Keratosen und hierbei insbesondere Akne und diabetischer Fuß durch topische Applikation von D₂O erfolgreich behandelt werden.

Eine bevorzugte Ausführungsform betrifft daher die erfindungsgemäße Verwendung von D₂O durch topische Applikation von D₂O auf die Haut, vorzugsweise auf die Haut eines tierischen Organismus, insbesondere eines Säugetieres, eines humanen oder nicht-humanen Säugetiers, wie beispielsweise Mensch, Ratte, Maus, Pferd, Schwein, Schaf und Ziege. Die topische Applikation erfolgt bevorzugt durch
1) direkte Applikation als Flüssigkeit oder Formulierung, insbesondere Salbe, Creme oder Gel (nachfolgend näher beschrieben);
2) Applikation über ein Pflaster oder eine Bandage (nachfolgend näher beschrieben) oder
3) Applikation als Aerosol (nachfolgend näher beschrieben).

Eine weitere bevorzugte Ausführungsform betrifft die erfindungsgemäße Verwendung von D₂O, wobei D₂O die Proliferation von Hautzellen hemmt und/oder inhibiert.

Unter "Hautzellen" im Sinne der vorliegenden Erfindung sind sämtliche proliferierenden Zellen der Haut zu verstehen, beispielsweise, jedoch nicht hierauf beschränkt, Keratinozyten, Epidermiszellen, Dermiszellen, Fibroblasten, Kollagenzellen, Bindegewebszellen und Melanozyten.

Der Begriff "topisch" bzw. "topische Applikation" oder "topische Verabreichung" oder "topische Anwendung" in Sinne der vorliegenden Erfindung bedeutet das lokale Auftragen oder Einbringen von Wirkstoffen, z.B. pharmazeutischen Wirkstoffen oder nicht-pharmazeutischen Wirkstoffen, auf die Haut, bevorzugt als Flüssigkeit, Gas oder Formulierung, insbesondere Salbe, Creme oder Gel. Im Gegensatz zur systemischen Verabreichung von Wirkstoffen erfolgt die Aufnahme der Wirkstoffe ohne Transport zum Wirkort über den Blutkreislauf und ist damit in der Regel nebenwirkungsärmer als die systemische Verabreichung, da hohe Wirkstoffkonzentrationen nur in dem lokal behandelten Gebiet der Haut vorliegen.

Unter dem Begriff "hemmen" bzw. "Hemmung" im vorliegenden Zusammenhang ist zu verstehen, dass die Proliferationsrate von hyperproliferativen Zelle verlangsamt und/oder herabgesetzt wird, bevorzugt bis zu ca. 5%, vorzugsweise bis zu ca. 10%, ebenfalls bevorzugt bis zu ca. 20%, stärker bevorzugt bis zu ca. 30%, ebenfalls stärker bevorzugt bis zu ca. 40%, noch stärker bevorzugt bis zu ca. 50%, am stärksten bevorzugt bis zu ca. 60% gegenüber der Proliferationsrate dieser hyperproliferativen Zellen ohne Verabreichung von D₂O. Der Begriff "inhibieren" oder "Inhibierung" im vorliegenden Zusammenhang bedeutet, dass die Proliferationsrate von hyperproliferativen Zellen, bevorzugt über 50%, ebenfalls bevorzugt bis zu ca. 60%, weiterhin bevorzugt bis zu ca. 65%, vorzugsweise bis zu ca. 70%, ebenfalls bevorzugt bis zu ca. 80%, stärker bevorzugt bis zu cm. 90%, ebenfalls stärker bevorzugt bis zu ca. 95%, noch stärker bevorzugt bis zu ca. 98%, am stärksten bevorzugt um bis zu 100% gegenüber der Proliferationsrate dieser hyperproliferativen Zellen ohne Verabreichung des D₂O verlangsamt bzw. herabgesetzt wird.

Während der Verwendung der D₂O-haltigen Zusammensetzungen der Erfindung, insbesondere bei der Therapie von Psoriasis, ist beobachtet worden, dass die Wirkung für den gesamten Beobachtungszeitraum von 4 Wochen nach Beendigung der Therapie anhielt. Daher betrifft eine bevorzugte Verwendung der Erfindung die Verabreichung, insbesondere die topische Applikation, von D₂O, D₂O-haltigen Zusammensetzungen und D₂O in Kombination mit einem oder mehreren weiteren Wirkstoffen in Intervallen, d.h. mit Behandlungspausen. Die Behandlungspausen betragen vorzugsweise 1 Woche oder mehr, vorzugsweise 2, 3, 4, 5, 6, 7, 8, 9, 10 oder mehr Wochen. Die Behandlungsdauer zwischen den Behandlungspausen kann variieren, vorzugsweise zwischen 1 Tag und 50 Tagen, insbesondere 2, 3, 4, 5, 6, 7, 8, 9, 10, 11, 12, 13, 14, 5, 16, 17, 8, 19, 20, 21, 22, 23, 24, 25, 26, 27, 28, 29, 30 oder mehr Tage. Die erste Behandlung wird vorzugsweise bis zum weitgehenden Rückgang der Symptome der jeweiligen hyperproliferativen Erkrankung durchgeführt. Die dafür notwendige Behandlungsdauer ist von der jeweils behandelten hyperproliferativen Erkrankung abhängig. Ob der Behandlungserfolg im gewünschten Maße eingetreten ist, wird von dem jeweils behandelnden Arzt entschieden. Beispielsweise liegt bei Psoriasis der Behandlungszeitraum bis zu einem weitgehenden Rückgang der Symptome zwischen 1 und 10 Tage, bei der Behandlung von Keratosen, insbesondere Akne und diabetischen Fuß zwischen 10 und 50 Tagen und bei der Behandlung von hypertrophen Narben zwischen 5 und 30 Tagen.

In einer offenbarten Ausführungsform der Therapie von Psoriasis wird D₂O, eine D₂O-haltige Zusammensetzung oder D₂O in Kombination mit einem oder mehreren weiteren Wirkstoffen gemäss der Ansprüche für einen Zeitraum von 1 bis 10 Tage, z.B. 1, 2, 3, 4, 5, 6, 7, 8, 9 oder 10 Tagen auf die betroffenen Hautareale appliziert. Diese Behandlung wird in Intervallen von 1 bis 10, z.B. 1, 2, 3, 4, 5, 6, 7, 8, 9, 10 Wochen wiederholt.

Mit der vorliegenden Erfindung wird darüber hinaus nicht nur die Wirkung von D₂O allein gezeigt, die Teilungsrate von hyperproliferativen Zellen in erkranktem Hautgewebe zu hemmen bzw. inhibieren, sondern auch, dass die Verabreichung einer Kombination aus D₂O mit einem anderen pharmazeutischen Wirkstoff gemäss der Ansprüche, diese Wirkung verstärken kann. Ebenfalls kann zusätzlich oder anstelle eines weiteren pharmazeutischen Wirkstoffs die Verwendung von D₂O zusammen mit einem weiteren nicht-pharmazeutischen Wirkstoff erfolgen, insbesondere zur Optimierung der topischen Applikation von D₂O als pharmazeutischen Wirkstoff auf der Haut. Eine solche Kombination aus D₂O und mindestens einem weiteren pharmazeutischen Wirkstoff und/oder mindestens einem weiteren nicht-pharmazeutischen Wirkstoff wird nachfolgend als "erfindungsgemäße Kombination" bezeichnet.

Konsequenterweise sind sämtliche in dieser Beschreibung offenbarten erfindungsgemäßen Verwendungen und (topisehen) Applikationen von D₂O ebenfalls auf eine erfindungsgemäße Kombination uneingeschränkt anwendbar, sofern nichts Gegenteiliges angezeigt ist. Ebenfalls finden Verwendungen einer erfindungsgemäßen Kombination in Bezug auf die erfindungsgemäßen Schichtsysteme, Mischungen aus D₂O und H₂O, Pflaster und Bandagen, Formulierungen und Aerosole (siehe unten) uneingeschränkt Anwendung sofern nichts Gegenteiliges angezeigt ist.

Eine bevorzugte Ausführungsform der vorliegenden Erfindung betrifft demnach die erfindungsgemäße Verwendung von D₂O, wobei das D₂O zusammen mit mindestens einem weiteren pharmazeutischen Wirkstoff und/oder mindestens einem weiteren nicht-pharmazeutischen Wirkstoff verwendet wird. Bevorzugt ist der mindestens eine weitere pharmazeutische Wirkstoff ausgewählt ist aus der Gruppe, bestehend aus Zytostatika, Proteinen, Peptiden, Nukleinsäuren, immunosuppressiven Wirkstoffen, wie Kortikoiden, und Wachstumsfaktoren. Der mindestens eine weitere nicht-pharmazeutische Wirkstoff ist bevorzugt ausgewählt ist aus der Gruppe, bestehend aus pharmazeutisch verträglichen anorganischen oder organischen Säuren oder Basen, Polymeren, Copolymeren, Block-Copolymeren, Einfachzucker, Mehrfachzucker, ionische und nicht-ionische Tenside oder Lipiden sowie Mischungen hiervon. Darüber hinaus zählen zu dieser Gruppe spezielle Proteine wie Albumin, Transferrin und Kinase-Inhibitoren. Das Ziel solcher Kombinationen aus D₂O und weiteren pharmazeutischen und/oder nicht-pharmazeutischen Wirkstoffen liegt in der Verstärkung der anti-proliferativen Wirkung und/oder der Verbesserung der topischen Applikation von D₂O auf der Haut.

Der hierin verwendete Begriff "Wirkstoff" umfasst alle pharmazeutischen bzw. nicht-pharmazeutischen Wirkstoffe der vorliegenden Erfindung. Der Begriff "pharmazeutischer Wirkstoff" wird hierin mit dem Begriff "Arzneimittel" synonym verwendet und bezeichnet im Sinne der vorliegenden Erfindung jede(s) beliebige anorganische oder organische Molekül, gemäss der Ansprüche Substanz oder Verbindung, das/die eine pharmakologische Wirkung aufweist. Die Wirkung eines erfindungsgemäßen pharmazeutischen Wirkstoffs ist eine anti-proliferative Wirkung auf Zellen der Haut, so dass es sich um "anti-proliferative pharmazeutische Wirkstoffe" oder auch "anti-proliferative Wirkstoffe" handelt. Als pharmazeutischer Wirkstoff gemäß der vorliegenden Erfindung ist auch D₂O anzusehen. Der Begriff "nicht-pharmazeutischer Wirkstoff" bezeichnet im Sinne der Erfindung jede(s) pharmakologisch verträgliche und therapeutisch sinnvolle Molekül, Substanz oder Verbindung, das/die kein pharmazeutischer Wirkstoff ist, jedoch vorzugsweise zusammen mit mindestens einem pharmazeutischen Wirkstoff an einen zu behandelnden Organismus verabreicht wird, beispielsweise in einer erfindungsgemäßen Formulierung formuliert, um qualitative Eigenschaften des/der pharmazeutischen Wirkstoffs/Wirkstoffe bzw. der erfindungsgemäßen Formulierung zu beeinflussen, insbesondere zu verbessern. Bevorzugt entfalten die nicht-pharmazeutischen Wirkstoffe keine oder im Hinblick auf die beabsichtigte Therapie keine nennenswerte oder zumindest keine unerwünschte pharmakologische Wirkung. Geeignete nicht-pharmazeutische Wirkstoffe sind beispielsweise pharmakologisch unbedenkliche Salze, beispielsweise Natriumchlorid, Geschmackstoffe, Vitamine, z.B. Vitamin A oder Vitamin E, Tocopherole oder ähnliche im menschlichen Organismus vorkommende Vitamine oder Provitamine, Antioxidantien, wie beispielsweise Ascorbinsäure, sowie Stabilisatoren und/oder Konservierungsstoffe zum Verlängern der Verwendungs- und Aufbewahrungsdauer eines pharmazeutischen Wirkstoffes oder einer erfindungsgemäßen Formulierung und sonstige dem Fachmann bekannte, übliche nicht-pharmazeutische Wirkstoffe bzw. Hilfs- und Zusatzstoffe. Weitere erfindungsgemäß bevorzugte nicht-pharmazeutische Wirkstoffe sind insbesondere alle zur Bildung wässriger Gele befähigten Stoffe, wie z.B. natürliche oder synthetische wasserlösliche Polymere, die Netzwerke ausbilden können.

Nachfolgend werden erfindungsgemäß bevorzugte weitere pharmazeutische Wirkstoffe einer erfindungsgemäßen Kombination und deren Wirkung aufgerührt, wobei diese Aufzählung nur beispielhaft ist und die vorliegende Erfindung nicht hierauf beschränkt ist:

### • Wasserlösliche zytotoxische Wirkstoffe

Durch Zugabe von wasserlöslichen zytotoxischen Wirkstoffen, insbesondere, aber nicht ausschließlich, von Zytostatika oder Mischungen mehrerer Zytostatika, kann eine Erhöhung der zytotoxischen Wirksamkeit im ausgewählten Hautbereich erreicht werden. Der Begriff "Zytostatika" bezeichnet natürliche oder chemische Substanzen oder Verbindungen, die in den Zellzyklus proliferativer Zellen eingreift, indem sie die Proliferation erheblich verzögern, hemmen oder inhibieren, beispielsweise durch Hemmung der DNA-Synthese mit kurzfristiger RNA- und Proteinsynthesehemmung. Durch Zugabe von beliebigen wasserlöslichen Zytostatika, die zur Behandlung hyperproliferativer maligner Erkrankungen der Haut geeignet sind, wie zum Beispiel, aber nicht hierauf beschränkt, Bleomycin, Cyclophosphamid, Doxorubicin, Paclitaxel (Taxol), Vincristin oder Mischungen mehrerer solcher Zytostatika, kann ebenfalls eine Nachhaltigkeit der D₂O-Wirkung im Sinne einer Reduktion der neoplastischen Bereiche in der Haut auch nach Beendigung der D₂O-Applikation erreicht werden. Gleichzeitig werden die Nebenwirkungen auf nicht zu behandelnde Organe, wie Leber und Niere, verringert, da D₂O gerichtet auf der Haut appliziert wird und nur in geringen Konzentrationen in das System gelangt. Eine systemische Verbreitung von D₂O und vorgenannten Wirkstoff(en) wird somit nahezu vollständig verhindert.

Beispiele für erfindungsgemäß geeignete Zytostatika sind (eingeteilt nach ihrem Wirkungsmechanismus):
- alkylierende und quervernetzende Zytostatika (sie schädigen die DNA): Cyclophosphamid, N-Nitrosoverbindungen, wie Carmustin, Ethylenimin-(Aziridin-)-Derivate, wie Thiotepa, Methansulfate, wie Busulfan, Platin-Komplexe, wie Cisplatin, und Procarbazin,
- Zytostatische Antibiotika: Anthracycline, wie Daunorubicin, Doxorubicin, Bleomycin und Mitomycine (letztgenannte interkalieren in DNA und hemmen Topoisomerasen), und
- Antimetabolite (sie verdrängen natürliche Stoffwechselbausteine): Folsäure-Antagonisten, wie Methotrexat, und Nucleosid-Analoga, wie Mercaptopurin, Fluorouracil.

Durch Applikation von Zytostatika, die mit anderen, vorzugsweise pharmazeutisch wirksamen, Molekülen (beispielsweise Polypeptide) konjugiert sind, kann die spezifische Wirksamkeit noch weiter verstärkt werden. Weitere Beispiele zur Verstärkung der spezifischen Wirksamkeit von Zytostatika sind Substanzen bzw. pharmazeutische Wirkstoffe aus der Gruppe der Antibiotika und der Chemotherapeutika.

### • Immunosuppressive Wirkstoffe

Durch Zugabe von Kortikoiden und/oder anderen immunosuppressiven Wirkstoffen oder Immunmodulatoren kann die Reaktion des Hautgewebes auf die D₂O-Behandlung, insbesondere bei bereits vorhandenen Entzündungen, verbessert und optimiert werden.

### • Wachstumsfaktoren

Durch Zugabe von Wachstumsfaktoren wie zum Beispiel, aber nicht hierauf beschränkt, TNF-alpha, TGF-beta, PDGF, IGF, Interleukin-4, Endothelin-1, CTGF und VEGF, kann die differentielle Wirkung des D₂O auf die hyperproliferierenden Zellen noch verstärkt werden. Während das D₂O bevorzugt in die hyperproliferierenden Bereiche des Hautgewebes gelangt und dort die Zellteilung hemmt oder inhibiert, verteilen sich die Wachstumsfaktoren homogen über normale und hyperproliferative Bereiche. In den normalen Bereichen können sie das Wachstum dieser Zellen bzw. Gewebe stimulieren, während ihre Wirkung in den hyperproliferativen Bereichen durch die Anwesenheit höherer D₂O-Konzentrationen weitgehend inhibiert wird. Damit lässt sich eine differential-therapeutische Strategie umsetzen, bei der gesunde Gewebe im Wachstum gefördert und kranke Zellen bzw. Gewebe im Wachstum inhibiert werden.

### • Nukleinsäuren

Durch Zugabe von Nukleinsäuren kann parallel zur zytotoxischen Wirkung des D₂O eine Veränderung der Erbinformation der Zellen im Bereich des Wirkortes oder ein gezieltes Ausschalten ("Gene Silencing") bestimmter Gene von Zellen im Bereich des Wirkortes innerhalb des Hautgewebes und dadurch eine Modifikation des Proteoms erreicht werden. Das "Gene Silencing" kann beispielsweise dazu führen, dass die in die DNA-Schadenabwehr involvierten Gene (zum Beispiel p53, BRCA1, BRCA2, ATM, CHK2) abgeschaltet werden und damit die durch D₂O an der Zellteilung gehinderten hyperproliferativen Zellen auch langfristig (nach Ende der topischen D₂O-Verabreichung) nicht mehr proliferieren, sondern schließlich durch Apoptose eliminiert werden. Methoden zum Durchführen eines "Gen Silencing" sind dem Fachmann gut bekannt und beispielsweise in Mello C C , Conte D "Revealing the world of DNA interference", in Nature 431, 338-342 (16. September 2004) beschrieben. Bevorzugt handelt es sich bei den Nukleinsäuren um DNA, vorzugsweise Oligonukleotide, Sense- oder Antisense-DNA, natürliche oder synthetische, DNA, genomische DNA, nackte DNA, einzel- oder doppelsträngige DNA, oder zirkuläre DNA, oder um RNA, vorzugsweise Antisense-RNA, RNAi, siRNA, oder andere zur Interferenz geeignete RNA-Moleküle, die in ihrer Länge nicht beschränkt sind.

Die Konzentration von erfindungsgemäß neben D₂O als pharmazeutischem Wirkstoff verwendeten weiteren pharmazeutischen Wirkstoffen bezogen auf die Gesamtlösung einer erfindungsgemäßen Kombination, liegt im Bereich von mindestens 10⁻⁸ M bis mindestens 5 x 10⁻² M, bevorzugt von mindestens 10⁻⁷ M bis 10⁻³ M, am stärksten bevorzugt von mindestens 10⁻⁶ M bis mindestens 10⁻² M. Ein insbesondere bevorzugter Konzentrationsbereich liegt im Bereich von mindestens 10⁻⁹ M bis mindestens 10⁻² M.

In einer besonders bevorzugten Ausführungsform umfassen die erfindungsgemäßen Wirkstoffkombinationen D₂O und Vitamin D Derivate, die als Agonisten des Vitamin D Rezeptors wirken, insbesondere Calcipotriol; Retinoid Derivate, die als Agonisten des Retinoid Rezeptors (RAR) wirken, insbesondere Tazarotene; Kortikosteroid-Derivate, die als Agonisten des Glukokortikoid-Rezeptors wirken, insbesondere Betamethason und Cortison; Fumarsäure, hautverdünnende Agenzien, insbesondere Clobetasol; Antagonisten der Dihydrofolat-Dehydrogenase beispielsweise Metothrexat und immunsuppressive Substanzen beispielsweise Amphotericin, Busulphan, Cotrimoxazol, Chloramburil, Colony Stimulating Faktor, Cyclophosphamid, Fluconazol, Ganciclovir, Methylprednisolon, Octreotid, Oxpentifyllin, Riluzol, Thalidomid, Zolimomab aritox. und Calcineurin-Antagonisten, insbesondere Cyclosporine, z.B. Cyclosporin A, Cyclosporin G, Cyclosporin B, Cyclosporin C, Cyclosporin D, Dihydro-cyclosporin D, Cyclosporin E, Cyclosporin F, Cyclosporin H, Cyclosporin I, Pimecrolimus, oder Tacrolimus.

Nachfolgend werden erfindungsgemäß bevorzugte weitere nicht-pharmazeutische Wirkstoffe einer erfindungsgemäßen Kombination und deren Wirkung sowie geeignete Konzentrationen aufgeführt, wobei diese Aufzählung nur beispielhaft ist und die vorliegende Erfindung nicht hierauf beschränkt ist:

### • Wasserlösliche Hilfs- und Zusatzstoffe

Durch Zugabe von wasserlöslichen Hilfs- und Zusatzstoffen, wie z.B. pharmazeutisch verträglichen anorganischen oder organischen Säuren, Basen, Salzen und/oder Puffersubstanzen zur Einstellung des pH Wertes, kann die physiologische Verträglichkeit des D₂O auf und in der Haut für normal proliferierende Zellen verbessert werden.

Beispiele für bevorzugte anorganische Säuren sind ausgewählt aus der Gruppe, bestehend aus Salzsäure, Bromwasserstoffsäure, Salpetersäure, Schwefelsäure und Phosphorsäure, wobei insbesondere Salzsäure und Schwefelsäure bevorzugt sind. Beispiele für besonders geeignete organische Säuren sind ausgewählt aus der Gruppe bestehend aus Äpfelsäure, Weinsäure, Maleinsäure, Bernsteinsäure, Essigsäure, Ameisensäure und Propionsäure und insbesondere bevorzugt Ascorbinsäure, Fumarsäure und Zitronensäure. Gegebenenfalls können auch Mischungen der genannten Säuren eingesetzt werden, insbesondere von Säuren, die neben ihren Säuerungseigensobaften auch andere Eigenschaften, z.B. in Verwendung als Geschmackstoffe oder Antioxidantien, besitzen, wie beispielsweise Zitronensäure oder Ascorbinsäure. Beispiele für pharmazeutisch verträgliche Basen sind Alkalihydroxide, Alkalicarbonate und Alkali-Ionen, bevorzugt Natrium. Mischungen dieser Substanzen können insbesondere zur Einstellung und Pufferung des pH Wertes benutzt werden, besonders bevorzugt sind hierbei Kaliumhydrogenphosphat und Di-Kaliumhydrogenphosphat sowie Natriumhydrogenphosphat und Di-Natriumhydrogenphosphat. Bevorzugte Puffersubstanzen im Sinne der Erfindung sind weiterhin PBS, HEPES, TRIS, MOPS sowie weitere physiologisch verträgliche Substanzen mit einem pK Wert im Bereich 5,0 bis 9,0. Die Konzentration dieser Substanzen, bezogen auf die Gesamtlösung einer erfindungsgemäßen Kombination, liegt vorzugsweise im Bereich von Mikromolar bis Millimolar, besonders bevorzugt im Bereich 1-100 mM.

### • Wasserlösliche, nicht-zytotoxische Moleküle

Durch Zugabe von wasserlöslichen, nicht-zytotoxischen Molekülen, wie z.B. bestimmten Polymeren (z.B., aber nicht hierauf beschränkt, Dextran, Polyethylenglykol, Agarose, Zellulose, Hylaronsäure), Copolymeren und Block-Copolymeren kann durch deren hohe Wasserbindungskapazität eine zusätzliche Verzögerung (Retardierung) des Übergangs des D₂O von topischen Applikationsform in die Haut, wie auch von der Haut ins System, erreicht werden. Durch die Fähigkeit der Polymere, das chemische Potential (Wasserpotential) von D₂O zu erniedrigen, kann darüber hinaus die Stärke und Richtung des osmotischen Gradienten über die Haut verändert bzw. verbessert und optimiert werden. Die Konzentration dieser Substanzen, bezogen auf die Gesamtlösung, liegt im Bereich von Mikromolar bis Molar, bevorzugt im Bereich 1- 500 mM.

### • Wasserlösliche, nicht-polymere Moleküle

Durch Zusatz von wasserlöslichen, nicht-polymeren Molekülen, welche die Dichte und/oder Viskosität von D₂O verändern, beispielsweise, aber nicht hierauf beschränkt, Einfachzucker und Mehrfachzucker, insbesondere Glukose, Sacharose, Dextrose, Maltose, Stärke und Zellulose, können die osmotischen Verhältnisse im Bereich der topischen D₂O Applikation sowie der D₂O-Transport und die D₂O Retention in der Haut verändert bzw. optimiert werden. Die Konzentration dieser Substanzen, bezogen auf die Gesamtlösung einer erfindungsgemäßen Kombination, liegt vorzugsweise im Bereich von Millimolar bis Molar, besonders bevorzugt im Bereich 1,0 mM bis 1,5 M.

### • D₂O-Grenzflächenspannungs-verändernde Substanzen

Durch Zugabe von Substanzen, welche die Grenzflächenspannung von D₂O verändern, beispielsweise, aber nicht hierauf beschränkt, ionische und nicht-ionische Tensiden oder Lipiden, insbesondere einer Mischung aus Tensiden und Lipiden, kann der Transport des D₂O von der topischen Applikation in die Haut sowie innerhalb der Haut verändert werden. Darüber hinaus können derartige Moleküle in Kombination mit den oben zu den pharmazeutischen Wirkstoffen aufgeführten wasserlöslichen zytetoxischen Wirkstoffen, vor allem Zytostatika, die Rolle eines Lösungsvermittlers für schwer in D₂O lösliche zytotoxische Wirkstoffe, insbesondere Zytostatika, übernehmen und damit unter anderem, aber nicht herauf beschränkt, den Transport höherer Mengen des zytotoxischen Wirkstoffes, insbesondere Zytostatikum, pro Dosis, insbesondere pro topischer Applikation (zu erfindungsgemäßen topischen Applikationen siehe nachfolgend) ermöglichen. Die Konzentration dieser Substanzen, bezogen auf die Gesamtlösung einer erfindungsgemäßen Kombination, liegt vorzugsweise im Bereich von Mikomolar bis Millimolar, besonders bevorzugt im Bereich 1- 500 mM.

### • Wasserlösliche Moleküle, die von stark proliferierenden (hyperpro-liferativen) Zellen bevorzugt aufgenommen werden

Durch Zugabe von wasserlöslichen Molekülen, welche dafür bekannt sind, dass sie durch stark proliferierende Zellen in besonderem Maße aufgenommen werden, beispielsweise Albumin oder Transferrin, kann eine zusätzliche Erhöhung der D₂O-Transportrate der von einer D₂O-Hydrathülle umgebenen Molekülen in die Targetzellen der Haut erreicht werden.

### • DNA-Reparatur-Moleküle

Durch Zugabe von Molekülen, die geeignet sind, die Reparatur und Wiederherstellung beschädigter DNA Stränge zu verhindern (insbesondere, aber nicht ausschließlich DNA-Reparatur-Proteine, wie Kinase-Inhibitoren) und/oder die Apoptose von Zellen mit beschädigter DNA zu induzieren, kann eine Nachhaltigkeit der D₂O-Wirkung auch nach dem Ende der Verabreichung von D₂O erreicht werden.

Die Konzentration bzw. Dosierung von D₂O und ggf. des mindestens einen weiteren pharmazeutischen und/oder nicht-pharmazeutischen Wirkstoffs unterliegt verschiedenen Faktoren, beispielsweise der Art der Behandlung, Art der Erkrankung, Erkrankungszustand des Patienten (Säugetier), Art des Wirkstoffs usw.. Dem Fachmann sind solche Parameter bekannt und die Bestimmung der speziellen Dosierungen unterliegt dem Fachwissen des Fachmanns. Geeignete Konzentrationsangaben werden hierin offenbart. Einige beispielhafte Angaben zu geeigneten Konzentrationsbereichen sind bereits oben aufgeführt, wobei diese lediglich Richtwerte darstellen sollen.

Das erfindungsgemäß verwendbare D₂O liegt bevorzugt als Flüssigkeit vor. Vorzugsweise liegt das D₂O in einer Lösung, bevorzugt H₂O (Wasser) als Lösungsmittel, vor und wird hierin auch als "Mischung aus D₂O und H₂O", wenn H₂O enthalten ist, bzw. als "D₂O-Lösung" oder "reines D₂O", wenn kein H₂O enthalten ist, bezeichnet. Eine erfindungsgemäße Mischung aus D₂O und H₂O enthält D₂O vorzugsweise in einem Konzentrationsbereich von 1 bis 99%, bevorzugt von 5 bis 98%, weiterhin bevorzugt von 10 bis 90%, ebenfalls bevorzugt von 15 bis 80%, stärker bevorzugt von 20 bis 70%, ebenfalls stärker bevorzugt von 30 bis 60%, am stärksten bevorzugt von 40 bis 50%, wobei sich diese Angaben auf den Gesamtwassergehalt der Mischung aus D₂O und H₂O beziehen. Die Herstellung einer erfindungsgemäßen D₂O-Lösung und analog hierzu einer erfindungsgemäßen Kombination erfolgt beispielsweise durch Mischen der Komponenten, insbesondere von D₂O und ggf. H₂O sowie ggf. des mindestens einen weiteren pharmazeutischen und/oder nicht-pharmazeutischen Wirkstoffs. Weiterhin kann ggf. ein Lösungsmittel, wie nachfolgend beschrieben, durch Mischen hinzugefügt werden. Vorzugsweise erfolgt die Beimischung von H₂O bzw. des mindestens einen weiteren pharmazeutischen und/oder nicht-pharmazeutischen Wirkstoffs bzw. des Lösungsmittels zu D₂O im flüssigen Aggregatzustand. Die Herstellung kann jedoch durch jedes beliebige geeignete Verfahren erreicht werden. Im Fall, dass D₂O allein (d.h. nicht in Kombination mit weiteren pharmazeutischen und/oder nicht-pharmazeutischen Wirkstoffen) und bei einer Konzentration von 100% bezogen auf das Gesamtvolumen der Lösung (d.h. reines D₂O) verwendet wird, stellt reines D₂O die erfindungsgemäße D₂O-Lösung dar.

Sämtliche in dieser Beschreibung offenbarten erfindungsgemäßen Verwendungen und (topischen) Applikationen von D₂O sind ebenfalls auf eine erfindungsgemäße Mischung aus D₂O und H₂O uneingeschränkt anwendbar, sofern nichts Gegenteiliges angezeigt ist. Ebenfalls finden Verwendungen einer erfindungsgemäßen Mischung aus D₂O und H₂O in Bezug auf die erfindungsgemäßen Kombinationen, Schichtsysteme, Pflaster und Bandage, Formulierungen und Aerosole (siehe unten) uneingesehränkt Anwendung sofern nichts Gegenteiliges angezeigt ist.

Die erfindungsgemäße Verwendung von D₂O kann, wie nachfolgend näher beschrieben, ebenfalls als Aerosol, Dampf oder Formulierung, insbesondere als Creme, Salbe oder Gel, erfolgen. Auf die in diesem Zusammenhang zu verwendenden Konzentrationen von D₂O wird ebenfalls nachfolgend eingefangen.

In einer bevorzugten Ausführungsform ist der mindestens eine weitere pharmazeutische Wirkstoff bzw. weitere nicht-pharmazeutische Wirkstoff an D₂O gebunden. "Gebunden" im Sinn der vorliegenden Erfindung bedeutet, dass der pharmazeutische bzw. nicht-pharmazeutische Wirkstoff von dem D₂O hydratisiert wird.

In weiteren bevorzugten Ausführungsformen ist das D₂O bzw. die erfindungsgemäße Kombination in einem geeigneten Lösungsmittel enthalten. Ein erfindungsgemäßes Lösungsmittel kann ein anorganisches oder organisches Lösungsmittel sein. Geeignete Lösungsmittel der vorliegenden Erfindung sollten vorzugsweise von dem Organismus (insbesondere Säugetier), dem der Wirkstoff mit Lösungsmittel verabreicht wird, physiologisch gut verträglich sein, d.h. keine Nebenwirkungen, z.B. toxische Nebenwirkungen, auslösen. Ein insbesondere bevorzugtes Lösungsmittel ist destilliertes Wasser. Ebenfalls bevorzugt sind Ethanol-Wasser-Mischungen; hierbei liegt der prozentuale Massenanteil von Ethanol in diesen Mischungen bevorzugt im Bereich zwischen 5 % und 99 % Ethanol, ebenfalls bevorzugt im Bereich von 10 % bis 96 % Ethanol, stärker bevorzugt zwischen 50 % und 92 %, am stärksten bevorzugt zwischen 69 % und 91 % Ethanol.

D₂O oder eine erfindungsgemäße Kombination können "vorformuliert" vorliegen, beispielsweise verpackt in geeigneten Mitteln zum Transport von pharmazeutischen Wirkstoffen, sog. "drug delivery"-Systemen, beispielsweise in Nanopartikeln, Vektoren, bevorzugt Gentransfer-Vektoren, viralen oder nicht viralen Vektoren, Poly- oder Lipoplex-Vektoren, Liposomen oder Hohlkolloide (d.h. Hohlkugeln kolloider Dimension). Weiterhin zum Transport geeignet sind nackte Nukleinsäuren, insbesondere nackte DNA. Geeigneten Vektoren, Liposomen, Hohlkolloide oder Nanopartikel sowie Verfahren zum Einbringen von Substanzen, in solche Vektoren, Liposomen, Hohlkolloide oder Nanopartikel sind allgemein im Stand der Technik gut bekannt und beispielsweise in Cryan S-A., Carrier-based strategies for Targeting Protein and Peptide Drugs to the Lungs, AAPS Journal, 2005, 07(01):E20-E41 und Sambrook et al. Molecular Cloning A Laboratory Manual, Cold Spring Harbor Laboratory (1989) NY beschrieben. Als Gentransfer-Vektoren können vorzugsweise Polyethylenimine oder kationische Lipide, wie z.B DOTAP, verwendet werden. Liposomen sind bevorzugt für die Verpackung von Zytostatika verwendbar; eine detaillierte Beschreibung erfolgt beispielsweise in Koshkina NV et al., Koshkina, N.V., et al., Paclitacel liposome aerosol treatment induces inhibition of pulmonary metastases in murine renal carcinoma model., Clinical Cancer Research, 2001, 7, 3258-3262. Proteine als pharmazeutische Wirkstoffe können vorzugsweise mittels superkritischer Flüssigkeiten, Emulsionsverfahren und Sprühtrocknung in biokompatible Poly-Mileh/Glykolsäure-Polymere (PLGA) verpackt werden.

Die topische Applikation von D₂O kann ebenfalls über ein Pflaster oder eine Bandage erfolgen. Eine weitere bevorzugte Ansführungsform betrifft demnach die erfindungsgemäße Verwendung von D₂O, wobei D₂O mit einem bzw. über ein Pflaster oder eine Bandage topisch appliziert wird. Bevorzugt kann das D₂O mit mindestens einem weiteren pharmazeutischen Wirkstoff und/oder mindestens einem weiteren nicht-pharmazeutischen Wirkstoff gemäß der vorliegenden Erfindung verwendet werden. Vorzugsweise ist der mindestens eine weitere pharmazeutische Wirkstoff ausgewählt ist aus der Gruppe, bestehend aus Zytostatika, Proteinen, Peptiden, Nukleinsäuren, immunosuppressiven Wukstoffen, wie Kortikoiden, und Wachstumsfaktoren. Der mindestens eine weitere nicht-pharmazeutische Wirkstoff ist bevorzugt ausgewählt ist aus der Gruppe, bestehend aus pharmazeutisch vertraglichen anorganischen oder organischen Säuren oder Basen, Polymeren, Copolymeren, Block-Copolymeren, Einfachzucker, Mehrfachzucker, ionische und nicht-ionische Tenside oder Lipiden sowie Mischungen hiervon Darüber hinaus zählen zu dieser Gruppe spezielle Proteine wie Albumin, Transferrin und Kinase-Inhibitoren.

Als "Pflaster" oder "Bandagen" im Sinne der Erfindung, sind alle auf der Haut durch mechanische oder chemische Wechselwirkung, Physisorption, Adhäsion oder andere physikalisch-chemische Prozesse fixierbare Vorrichtungen zu verstehen, welche dazu geeignet sind, einen ausgewählten Hautbereich okklusiv oder nicht-okklusiv über einen für die beabsichtigte Behandlung angemessen langen Zeitraum abzudecken und die Zuführung von D₂O an die Haut zu ermöglichen und/oder unterstützen. Erfindungsgemäß anwendbare Pflaster und Bandagen als Applikationssysteme zur lokalen Freisetzung von Wirkstoffen auf der Haut (z B. Wärmepflaster) sowie zur kontrollierten systemischen Freisetzung von Wirkstoffen (z B Opiat-Depotpflaster, Nitroglycerin-Depotpflaster) sind im Stand der Technik bekannt. Als "Depot-Pflaster" oder "Depot-Bandage" soll zusätzlich zu den oben beschriebenen Eigenschaften die Fähigkeit des Pflasters oder der Bandage zur Speicherung von D₂O und deren kontrollierte Abgabe an die Haut über einen Zeitraum von Tagen oder Wochen verstanden werden Solche Depot-Pflaster bzw. Depot-Bandagen sind nachfolgend von den Begriffen Pflaster bzw Bandage umfasst.

Eine bevorzugte. Ausführungsform der vorliegenden Erfindung ist demnach ein Pflaster oder Bandage zur topischen Applikation, enthaltend D₂O. Ein(e) solche(s) erfindungsgemäße(s) Pflaster oder Bandage kann vorzugsweise weiterhin mindestens einen weiterer pharmazeutischer Wirkstoff und/oder mindestens einen weiteren nicht-pharmazeutischer Wirkstoff enthält Vorzugsweise ist der mindestens eine weitere pharmazeutische Wirkstoff ausgewählt ist aus der Gruppe, bestehend aus Zytostatika, Proteinen, Peptiden, Nukleinsäuren, immunosuppressiven Wirkstoffen, wie Kortikoiden, und Wachstumsfaktoren. Der mindestens eine weitere nichtpharmazeutische Wirkstoff ist bevorzugt ausgewählt ist aus der Gruppe, bestehend aus pharmazeutisch verträglichen anorganischen oder organischen Säuren oder Basen; Polymeren, Copolymeren, Block-Copolymeren, Einfachzucker, Mehrfachzucker, ionische und nicht-ionische Tenside oder Lipiden sowie Mischungen hiervon. Darüber hinaus zählen zu dieser Gruppe spezielle Proteine wie Albumin, Transferrin und Kinase-Inhibitoren.

Ein erfindungsgemäßes Pflaster oder eine erfindungsgemäße Bandage kann ebenfalls bevorzugt eine Mischung aus D₂O und H₂O enthalten. Auch ein(e) solches Pflaster oder Bandage kann bevorzugt weiterhin mindestens einen weiteren pharmazeutischen und/oder mindestens einen weiteren nicht-pharmazeutischen Wirkstoff der Erfindung, wie vorstehend aufgeführt, enthalten.

Ein erfindungsgemäßes Pflaster oder eine erfindungsgemäße Bandage dient der topischen Applikation von D₂O auf die Haut. Das Pflaster oder die Bandage umfasst D₂O vorzugsweise in einer Anordnung, welche das D₂O in Form eines Depots speichert und dessen kontrollierte Abgabe an die Haut ermöglicht.

Insgesamt ist für jede kontrollierte Freisetzung von D₂O an die Haut folgendes Problem zu beachten: die Freisetzung aus einer direkt auf die Haut aufgetsagenen Flüssigkeit oder Formulierung als Salben, Creme oder Gel kann durch die Richtung des osmotischen Gradienten innerhalb der Haut von innen nach außen erschwert werden, da das D₂O in der Flüssigkeit, Salbe, Creme oder in dem Gel unter Umständen ein niedrigeres Wasserpotential aufweist als das H₂O in der Haut und in den darunter liegenden Gefäßen.

Eine besonders bevorzugte Ausführungsform der topischen Applikation von D₂O ist es daher, durch gezielte Manipulation der osmotischen Verhältnisse im zu behandelnden Hautgebiet die Tiefe bzw. den Grad des Eindringens von D₂O in die Haut zu regulieren und damit zu kontrollieren, und zwar vorzugsweise bis zu der Tiefe, in der die hyperproliferierenden Zellen liegen, bevorzugt bis zur Epidermis oder bis zur Dermis. Dies kann durch die gewählte Zusammensetzung einer apptizierten erfindungsgemäßen Kombination erreicht werden, indem Substanzen zugefügt werden, welche in der Lage sind, die osmotischen Verhältnisse, an der Oberfläche der Haut zu verändern. Beispiele für solche Substanzen sind weiter oben aufgeführt.

Eine weitere Möglichkeit zum kontrollierten Eindringen von D₂O in die Haut besteht in der Verwendung von einer oder mehreren Membran(en) oder Folie(n), welche die Passage von Wasser und Gasen ermöglicht / ermöglichen, von größeren Molekülen oder Partikeln (einschl. Bakterien, Viren, Einzeller) jedoch verhindert / verhindern. Beispiele für solche erfindungsgemäß verwendbaren Membranen und Folien sind im Stand der Technik bekannt und haben zahlreiche Anwendungen, wie z.B. in Textilien unter dem Markennamen GORE Tex® oder in der Medizin sogenannte Biofilme.

Eine ganz besonders bevorzugte Ausführungsform der Erfindung besteht deshalb in der erfindungsgemäßen Verwendung eines Pflasters oder einer Bandage, wobei das Pflaster oder die Bandage in Kombination mit mindestens einer Membran oder mindestens einer Folie verwendet wird. Vorzugsweise ist die mindestens eine Membran bzw. die mindestens eine Folie eine mikro- oder nanoporöse Membran bzw. Folie.

Ebenfalls von der vorliegenden Erfindung umfasst ist eine Membran oder Folie, die schon für sich genommen die Funktion eines Pflasters oder einer Bandage der Erfindung hat, und somit eine an sich eine fixierbare, bevorzugt selbstklebende, Vorrichtung zur okkulsiven oder nichtokkulsiven Abdeckung definierter Hautgebiete darstellt. Beispiele hierfür sind hypoallergene nano-oder mikroporöse "Tapestreifen" bzw. Pflaster zur Abdeckung von Wunden (z.B. Tegaderm®).

In einer besonders bevorzugten Ausführungsform wird D₂O über eine spezielle Anordnung topisch auf die Haut appliziert. Diese Anordnung besteht aus folgenden Komponenten:
- einer mikro- oder nanoporösen Membran oder Folie, welche direkt auf die Haut aufgebracht wird,
- gefolgt von einer D₂O-Schicht, welche das D₂O enthält,
- ggf. gefolgt von einer sog. Okklusionssehicht welche eine Verdampfung des D₂O nach außen verhindert oder reguliert und gleichzeitig einen mechanischen Schutz darstellt, welcher das Entweichen von D₂O als Flüssigkeit verhindert und
- einem Pflaster oder einer Bandage.

In einer weiteren bevorzugten Ausführungsform wird D₂O über eine spezielle Anordnung topisch auf die Haut appliziert. Diese Anordnung besteht aus folgenden Komponenten:
- eine vorstehend beschriebene mikro- oder nanoporösen Membran oder Folie, die schon für
   sich die Funktion eines Pflasters oder einer Bandage der Erfindung hat, welche direkt auf die Haut-aufgebracht wird,
- gefolgt von einer D₂O-Schicht, welche das D₂O enthält,
- ggf. gefolgt von einer sog Okklusionsschicht welche eine Verdampfung des D₂O nach außen verhindert oder reguliert und gleichzeitig einen mechanischen Schutz darstellt, welcher das Entweichen von D₂O als Flüssigkeit verhindert und
- einer weiteren mikro- oder nanoporösen Membran oder Folie, die schon für sich die Funktion eines Pflasters oder einer Bandage der Erfindung hat

Bei Bedarf können selbstverständlich weitere D₂O-Schichten hinzugefügt werden, die weiteres D₂O enthalten, und die von Membranen bzw. Folien separiert werden, wodurch die Depotwirkung oder der Übergang des D₂O in die Haut verändert werden kann, beispielsweise die Freisetzungsmenge und/oder -dauer von D₂O Der verwendete Begriff "D₂O-Schicht" bezieht sich auf flüssiges reines D₂O, eine erfindungsgemäße Mischung aus D₂O und H₂O und eine Formulierung von D₂O, insbesondere als Creme, Salbe oder Gel.

Ebenfalls können vorzugsweise auch Schichten hinzugefügt werden, die chemische, elektrische oder thermische Eigenschaften aufweisen, welche geeignet sind, den Übergang des D₂O in die Haut und/oder die Dauer seiner Freisetzung zu manipulieren. Beispiele hierfür sind Schichten, die geeignet sind, ein elektrisches, thermo-elektrisches, thermisches ader chemisches Potential (oder eine Kombination davon) über die darunter liegenden Schichten und die Haut aufzubauen und/oder aufrechtzuerhalten. Dies kann beispielsweise durch in erfindungsgemäße Membranen oder Folien eingebettete oder auf ihnen befindliche Elektroden erreicht werden, welche von außen mit Strom (Gleichspannung, Wechselspannung oder hochfrequente Ströme) versorgt werden oder die durch die gezielte Wahl des Elektrodenmaterials mit der D₂O Schicht als Elektrolyt elektrochemische Potentiale erzeugen

Die Gesamtheit solcher vorbeschriebenen D₂O-Schichten, Okkulsionsschichen; Schichten mit chemischen, elektrischen oder thermischen Eigenschaften, Membranen und Folien in jeder beliebigen, für den Verwendungszweck geeigneten Anzahl, Kombination und Anordnung, wird erfindungsgemäß als "Schichtsystem" bezeichnet. Ein solches Schichtsystem wird vorzugsweise in Verbindung mit einem/einer vorstehend beschriebenen (Depot-) Pflaster oder (Depot-) Bandage verwendet werden.

Durch Variation von Morphologie (Porengröße, Membran- bzw Foliendicke, Oberflächenrauheit und Oberflächenprofil) und Oberflächeneigenschaften (z B. hydrophil oder hydrophob, chemischen Beschichtungen -kovalent gebunden oder adhäsiv gebunden-, funktionellen Gruppen, Bindung oder Einlagerung anorganischer oder organischer Substanzen) der direkt im Kontakt mit der Haut befindlichen Membran oder Folie kann der Übergang des D₂O aus einem / einer erfindungsgemäßen Pflaster, Bandage oder Schichtsystem in die Haut gezielt beeinflusst bzw. verändert werden.

Eine weitere Variation des Eintritts von D₂O in die Haut ist durch die gezielte Verwendung von Klebstoffen möglich, welche zur mechanischen Befestigung des (Depot-) Pflasters oder der (Depot-) Bandage auf der Haut verwendet werden können, aber nicht zwingend notwendig sind. Die allgemein für topische Anwendungen von Pflastern und Bandagen verwendeten Klebstoffe weisen eher hydrophoben Charakter auf, welcher den Durchtritt von D₂O durch die Klebstoffschicht verhindert kann. Durch Zumischen von Zusatzstoffen in die Klebstoffzubereitung kann eine Veränderung dieser Eigenschaften erreicht werden. Als solche "Zusatzstoffe" kommen organische und/oder anorganische Substanzen und Verbindungen in Betracht, welche in der Lage sind, die Permeationseigenschaften von D₂O durch die Klebstoffschicht zu verändern. Beispiele für solche Substanzen sind u.a. Polymere, Co-Polymere, Blockpolymere, Block-Co-Polymere, Tenside, Peptide, Proteine, Nukleinsäuren, Sterote und Steroide.

In einer weiteren bevorzugten Ausführungsform wird D₂O, vorzugsweise mit einem Pflaster oder einer Bandage der Erfindung, über eine Anordnung topisch auf die Haut appliziert, die den Übergang von D₂O in die Haut weitgehend oder ausschließlich über die Dampfphase ermöglicht. D.h. erfindungsgemäß als Flüssigkeit verwendetes D₂O verdampft als molekulare D₂O und kommt als Dampf mit der Haut in Kontakt. Die Konzentrationen des D₂O entsprechen damit den oben beschriebenen Konzentrationen der erfindungsgemäßen (D₂O-enthaltenden) Flüssigkeit. Dampfförmiges D₂O hat den Vorteil eines besonders leichten Eindringens in die Haut. Um dieses Verdampfen zu bewirken, ist thermische Energie erforderlich, die entweder von der Haut selbst oder von einer externen Wärmequelle, z.B. bei Verwendung eines Pflasters oder einer Bandage der vorliegenden Erfindung einer in das oben beschriebene erfindungsgemäße Schichtsystem eingebrachten elektrischen Heizung (z.B. Peletier-Heizung), bezogen werden kann. Zusätzlich zu dieser thermischen Energie kann durch gezielte Modifikationen, beispielsweise der Wahl der Morphologie (insbesondere Porengröße und Oberflächenbeschichtung), einer ersten, auf der Haut befindlichen erfindungsgemäßen Membran oder Folie des erfindungsgemäßen Schichtsystems erreicht werden, dass nur dampfförmiges D₂O durch die bis zur Haut vordringen kann, flüssiges D₂O hingegen zurückgehalten wird. Auch bei dieser Ausführungsform lassen sich durch die oben beschriebenen Veränderungen des erfindungsgemäßen Schichtsystems und entsprechenden Modifikationen an ggf. weiteren verwendeten Membranen oder Folien, die Menge und die Dauer der Freisetzung von D₂O über die Dampfphase kontrolliert und/oder verändert werden.

Eine weitere bevorzugte Ausführungsform der Erfindung betrifft die erfindungsgemäße Verwendung von D₂O, wobei das D₂O als Aerosol appliziert wird. Die Applikation erfolgt vorzugsweise direkt auf die Haut.

Unter einem "Aerosol" versteht man feste oder flüssige schwebende Partikel mit einem Durchmesser von ca. 0,0001 µm bis ca. 100 µm, in Gasen insbesondere Luft, wobei Zusammensetzung und Form der Aerosole sehr stark variieren kann. Die kleinsten pharmazeutisch wirksamen Partikel in Aerosolen sind beispielsweise Nukleinsäuren, Peptide oder Proteine, die größten Partikel beispielsweise Nebelpartikel. Häufig bestehen Aerosole aus Gemischen von Partikeln unterschiedlicher Partikelgrößen und verkörpern dadurch eine polydisperse Größenverteilung. Aerosole lassen sich künstlich durch im Stand der Technik gut bekannte Dispersions- und Kondensationsverfahren herstellen. Sie können ohne Treibgas verwendet werden oder in Verbindung mit einem verflüssigten Druckgas als Treibgas, beispielsweise in Sprühdosen, verwendet werden.

Die Applikation eines erfindungsgemäßen Aerosols erfolgt bevorzugt über einen Vernebler. Als "Vernebler" für die vorliegende Erfindung ist jeder beliebige für medizinische Aerosole geeignete Apparat zu verstehen, mit dem Aerosolpartikel im Größenbereich 50 nm bis 50 µm produziert werden können. Erfindungsgemäß wird D₂O dem Vernebler zugeführt, um hieraus erfindungsgemäße, bevorzugt treibgasfreie, Aerosole herzustellen. Der Vernebler versprüht hierzu ein definiertes Volumen des D₂O, meist unter Anwendung hoher Drücke durch kleine Düsen, um so ein auf der Haut applizierbares, erfindungsgemäßes Aerosol zu erzeugen.

Geeignete Vernebler für erfindungsgemäße Aerosole umfassen auch Treibgas-getriebene Inhalationsgeräte bzw. Vernebler. Treibgase können hierbei beispielsweise FCKW oder HFKW sein. Diesbezüglich wird auf "Theorie und Praxis der Inhalationstherapie", Seiten 31 bis 70Arcis Verlag (2000) verwiesen, wo eine ausführliche Beschreibung verwendbarer Vernebler sowie Verfahren zu deren Anwendung offenbart ist/sind.

Beispiele für erfindungsgemäß geeignete Vernebler sind pressluftgetriebene Düsenvernebler (z.B. PARI LC plus, PARI GmbH, Starnberg, Deutschland), Venturi-Düsenvernebler, Wasserdampfgetriebene Düsenvernebler oder Ultraschallvernebler (z.B. AeronebLab, Aerogen, Inc., Stierlin Court, Canada; eFLOW, PARI GmbH, Starnberg, Deutschland). Ebenfalls geeignet sind Vernebler mit einer Größe, die von dem Patienten (Mensch) mitgeführt werden können, z.B. der Respimat@, wie in WO 97/12687, beschrieben. Sämtliche hierin zitierten Referenzen sind vollumfänglich in die vorliegende Erfindung einbezogen.

Ein weiterer Gegenstand der Erfindung betrifft ein Aerosol, welches eine Mischung aus D₂O und H₂O-enthält, zur Applikation auf die Haut.

Die Herstellung eines erfindungsgemäßen Aerosols sowie einer erfindungsgemäßen D₂O-Lösung zur Applikation als Aerosol kann anhand geeigneter bekannter Standardtechniken zur Aerosolherstellung erfolgen.

Die in einem erfindungsgemäßen Aerosol zu verwendenden Konzentrationen von D₂O ist von verschiedenen Faktoren abhängig, beispielsweise dem Verwendungszweck, Erkrankungszustand und unterliegen dem Fachwissen des Fachmanns. Ein erfindungsgemäßes Aerosol enthält D₂O vorzugsweise in einem Konzentrationsbereich von 5 bis 98 Gew:-%, bevorzugt von 10 bis 90 Gew.-%, ebenfalls bevorzugt von 15 bis 80 Gew.-%, stärker bevorzugt von 20 bis 70 Gew.-%, ebenfalls stärker bevorzugt von 30 bis 60 Gew.-%, am stärksten bevorzugt von 40 bis 50 Gew.-%.

Die erzeugten erfindungsgemäßen Aerosole, die D₂O allein (reines D₂O), eine Mischung aus D₂O oder H₂O oder eine erfindungsgemäße Kombination enthalten, werden unmittelbar auf die Haut im zu behandelnden Gebiet appliziert. Dies kann z.B. durch eine auf der Haut aufsetzbare und zu dieser hin geöffneten Kammer erfolgen, durch welche das Aerosol geleitet wird.

In einer weiteren bevorzugten Ausführungsform ist die Mischung aus D₂O und H₂O eines erfindungsgemäßen Aerosols sowie der ggf. enthaltenen weiteren pharmazeutischen und/oder nicht-pharmazeutischen Wirkstoff der Erfindung in einem Lösungsmittel enthalten, bevorzugt in mindestens einem anorganischen oder organischen Lösungsmittel enthalten. Bevorzugt handelt es sich um ein Lösungsmittel, ausgewählt aus der Gruppe, bestehend aus Ethanol, Wasser und Glycerol sowie Mischungen davon.

Sämtliche in dieser Beschreibung offenbarten erfindungsgemäßen Verwendungen und (topischen) Applikationen von D₂O finden ebenfalls auf ein erfindungsgemäßes Aerosol uneingeschränkt Anwendung, sofern nichts Gegenteiliges angezeigt ist. Ebenfalls finden Verwendungen eines erfindungsgemäßen Aerosols in Bezug auf eine erfindungsgemäße Kombination, erfindungsgemäße Mischung aus D₂O und H₂O, sowie eine erfindungsgemäße Formulierung uneingeschränkt Anwendung, sofern nichts Gegenteiliges angezeigt ist bzw. die beschriebene Verwendung für eine Verwendung der Komponenten in gasförmigem Zustand (als Aerosol) geeignet ist.

Eine weitere bevorzugte Ausführungsform betrifft die erfindungsgemäße Verwendung von D₂O, wobei D₂O als Formulierung topisch appliziert wird.

Ein weiterer Gegenstand der vorliegenden Erfindung betrifft eine Formulierung zur topischen Applikation auf Haut, enthaltend D₂O. Vorzugsweise ist die Formulierung eine Salbe, Creme oder Gel.

Unter einer "Salbe" gemäß der vorliegenden Erfindung ist eine äußerlich zu verwendende Arzneimittelzubereitung aus einer Grundmasse schrnierfähiger Stoffe, wie Vaseline, zu der die eigentlichen pharmazeutischen und/oder nicht pharmazeutische Wirkstoffe zugegeben werden, beispielsweise durch Mischen.

Unter einer "Creme" im Sinne der vorliegenden Erfindung ist eine erfindungsgemäße Salbe zu verstehen, die zusätzlich weitere Bestandteile enthalten kann, wie kosmetische Wirkstoffe, z.B. Duftstoffe, Farbstoffe und/oder Emulgatoren, z.B. Lecithin. Von einer Creme wird im Allgemeinen eine Lotion unterschieden, wobei diese Unterscheidung meistens abhängig von dem Grad der Viskosität gemacht wird. Erfindungsgemäß ist unter einer Creme jedoch auch eine Lotion zu verstehen.

Ein "Gel" der vorliegenden Erfindung ist die Lösung einer makromolekularen Substanz, z. B. Agarose oder Acrylamid, deren Konzentration so hoch ist, dass sich die gelösten Makromoleküle zu einem schwammartigen, dreidimensionalen Gerüst verbinden, in dessen Hohlräumen eine Flüssigkeit befindet. Dadurch haben Gele eine relativ feste Konsistenz. Die Viskosität liegt zwischen flüssig und fest. Eine solche Flüssigkeit ist bevorzugt reines D₂O oder eine erfindungsgemäße Mischung aus D₂O und H₂O.

In einer bevorzugten Ausführungsform enthält die erfindungsgemäße Formulierung, wobei die Formulierung mindestens einen weiteren pharmazeutischen Wirkstoff und/oder mindestens einen weiteren nicht-pharmazeutischen Wirkstoff. Der mindestens eine weitere pharmazeutische Wirkstoff ist vorzugsweise ausgewählt aus der Gruppe, bestehend aus Zytostätika, Proteinen, Peptiden, Nukleinsäuren, immunosuppressiven Wirkstoffen, wie Kortikoiden, und Wachstumsfaktoren. Der mindestens eine weitere nicht-pharmazeutische Wirkstoff ist bevorzugt ausgewählt aus der Gruppe, bestehend aus pharmazeutisch verträgliche anorganischen oder organischen Säuren oder Basen, Polymeren, Copolymeren, Block-Copolymeren, Einfachzucker, Mehrfachzucker, ionische und nicht-ionische Tenside oder Lipiden sowie Mischungen hiervon, Albumin, Transferrin und DNA-Reparatur-Proteinen, wie Kinase-Inhibitoren

Die Herstellung einer erimdungsgemäßen Formulierung, insbesondere einer Salbe, Creme oder einem Gel, ist exemplarisch m den Beispielen beschrieben. Sofern eine solche Formulierung weitere pharmazeutische und/oder nicht-pharmazeutische Wirkstoffe enthalten, werden diese vorzugsweise durch Mischen der Formulierung hinzugefügt. Sie kann jedoch nach jedem beliebigen im Stand der Technik bekannten Standardverfahren erfolgen. Dem Fachmann sind solche Verfahren und ebenfalls die zu wählenden Konzentrationen der zu verwendenden Komponenten bzw Substanzen bekannt.

Die Konzentrationen von D₂O m einer erfindungsgemäßen Formulierung liegen vorzugsweise in folgenden Bereichen.
- für eine Creme oder Salbe bevorzugt im Bereich von 2 bis 90 Gew. -%, bevorzugt von 5 bis 85 Gew.-%, ebenfalls bevorzugt von 10 bis 80 Gew -%, besonders bevorzugt von 15 bis 70 Gew. %, stärker bevorzugt von 20 bis 60 Gew. -% und am stärksten bevorzugt von 25 bis 50 Gew.-% und
- für ein Gel bevorzugt 5 bis 99 Gew -%, bevorzugt von 10 bis 90 Gew. -%, ebenfalls bevorzugt von 15 bis 80 Gew.-%, besonders bevorzugt von 20 bis 70 Gew.-%, starker bevorzugt von 30 bis 70 Gew -% und am stärksten bevorzugt von 35 bis 65 Gew.-%.

Der Fachmann wird insbesondere abhängig von der vorliegenden Indikation, dem Zustand des Patienten, der Schwere der Erkrankung usw die geeignete Konzentration wählen.

In einer besonders bevorzugten Ausführungsform enthält eine erfindungsgemäße Formulierung weiterhin mindestens ein anorganisches oder organisches Lösungsmittel. Bevorzugt ist das Lösungsmittel ausgewählt ist aus der Gruppe, bestehend aus Ethanol, Wasser und Glycerol sowie Mischungen davon

Sämtliche in dieser Beschreibung offenbarten erfindungsgemäßen Verwendungen und (topischen) Applikationen von D₂O finden ebenfalls auf die erfindungsgemäßen Formulierungen uneingeschränkt Anwendung, sofern nichts Gegenteiliges angezeigt ist. Ebenfalls finden Verwendungen einer erfindungsgemäßen Formulierung in Bezug auf die erfindungsgemäßen Schichtsysteme, Mischung aus D₂O und H₂O, Pflastern und Bandagen, Kombinationen und Aerosole uneingeschränkt Anwendung, sofern nichts Gegenteiliges angezeigt ist.

Die Formulierung der Erfindung enthält bevorzugt weiterhin mindestens ein anorganisches oder organisches Lösungsmittel, vorzugsweise ausgewählt aus der Gruppe, bestehend aus Ethanol, Wasser und Glycerol sowie Mischungen davon.

Die erfindungsgemäßen Formulierungen werden vorzugsweise topisch verabreicht. Die Herstellung einer Formulierung, enthaltene D₂O, oder eine erfindungsgemäße Kombination, sowie die Herstellung eines erfindungsgemäßen Aerosols enthaltend eine Formulierung, kann analog zu den Vorgehensweisen erfolgen, wie sie vorstehend für die Herstellung einer D₂O-Lösung, einer erfindungsgemäßen Kombination bzw. eines erfindungsgemäßen Aerosols beschrieben ist. Die Wahl und Konzentration der ggf. eingeschossenen Hilfs- und Zusatzstoffe hängt von dem Verwendungszweck der Formulierung ab und obliegt dem Fachwissen eines Fachmanns. Eine erfindungsgemäße Formulierung kann als Flüssigkeit Salbe, Creme oder Gel zubereitet werden. Die Herstellung solcher Flüssigkeiten, Salben, Cremes oder Gelen kann analog zu der obigen Beschreibung erfolgen. Verfahren hierfür sind im Stand der Technik bekannt. Konzentrationen der einzelnen Komponenten bzw. Substanzen sind von dem jeweiligen Verwendungszweck, Erkrankungszustand etc. abhängig und unterliegen dem Fachwissen des Fachmanns.

Weitere besonders bevorzugte Ausführungsformen der vorliegenden Erfindung betreffen die erfindungsgemäße Verwendung und Applikation von D₂O als erfindungsgemäßes Aerosol bzw. als erfindungsgemäße Formulierung zusammen mit mindestens einem weiteren pharmazeutischen Wirkstoff und/oder mindestens einem weiteren nicht-pharmazeutischen Wirkstoff. Ebenfalls besonders bevorzugte Ausführungsformen der vorliegenden Erfindung betreffen ein erfindungsgemäßes Aerosol bzw. eine erfindungsgemäße Formulierung, welches/welche mindestens einem weiteren pharmazeutischen Wirkstoff und/oder mindestens einem weiteren nicht-pharmazeutischen Wirkstoff enthält, wie hierin beschrieben. Bevorzugt ist der mindestens eine weitere pharmazeutische Wirkstoff ausgewählt ist aus der Gruppe, bestehend aus Zytostatika, Proteinen, Peptiden, Nukleinsäuren, immunosuppressiven Wirkstoffen, wie Kortikoiden, und Wachstumsfaktoren. Der mindestens eine weitere nicht-pharmazeutische Wirkstoff ist bevorzugt ausgewählt ist aus der Gruppe, bestehend aus pharmazeutisch verträglichen anorganischen oder organischen Säuren oder Basen, Polymeren, Copolymeren, Block-Copelymeren, Einfachzucker, Mehrfachzucker, ionische und nicht-ionische Tenside-oder Lipiden sowie Mischungen hiervon. Darüber hinaus zählen zu dieser Gruppe spezielle Proteine wie Albumin, Transferrin und Kinase-Inhibitoren.

Die vorliegende Erfindung wird nachfolgend anhand von Beispielen weiter erläutert.

### Beschreibung der Figuren

### Figur 1:

Mittlere Anzahl von Papeln und Papulopusteln in einen quadratischen, 10 x 10 cm großen Hautgebiet auf dem oberen Rückenbereich von 32 Testpersonen mit mittelschwerer Akne Vulgaris als Funktion der Bebandlungszeit (Versuchstage) mit einem D₂O Hydrogel (runde Symbole) und mit einem H₂O Hydrogel (quadratische Symbole). Die Zählung der entzündeten Follikel erfolgte alle 5 Tage ab Versuchbeginn, der Versuch erstreckte sich insgesamt über 40 Tage. Bei jeder Testperson wurde immer dieselbe Stelle (wasserfest auf der Haut markiert) ausgewertet. Jede Testperson erhielt sowohl das D₂O Hydrogel wie auch das H₂O Hydrogel an räumlich hinreichend getrennten und wasserfest markierten Bereichen des oberen Rückenbereichs aufgetragen, welche die zur Zählung verwendeten Bereiche umfassten.

### Figur 2:

Mittlere Anzahl von Komedonen in einen quadratische, 10 x 10 cm großen Hautgebiet auf dem oberen Rückenbereich von 32 Testpersonen mit mittelschwerer Akne Vulgaris als Funktion der Behandlungszeit (Versuchstage) mit einem D₂O Hydrogel (runde Symbole) und mit einem H₂O Hydrogel (quadratische Symbole). Die Zählung der nicht-entzündeten Follikel erfolgte alle 5 Tage ab Versuchbeginn, der Versuch erstreckte sich insgesamt über 40 Tage. Bei jeder Testperson wurde immer dieselbe Stelle (wasserfest auf der Haut markiert) ausgewertet Jede Testperson erhielt sowohl das D₂O Hydrogel wie auch das H₂O Hydrogel an räumlich hinreichend getrennten und wasserfest markierten Bereichen des oberen Rückenbereichs aufgetragen, welche die zur Zählung verwendeten Bereiche umfassten.

### Figur 3:

Mittlere Anzahl von Papeln und Papulopusteln in einen quadratischen, 10 x10 cm großen Hautgebiet auf dem oberen Rückenbereich von 14 Testpersonen mit mittelschwerer Akne Vulgaris als Funktion der Behandlungszeit (Versuchstage) mit einer D₂O Creme (runde Symbole) und mit einer H₂O Creme (quadratische Symbole). Die Zählung der entzündeten Follikel erfolgte alle 5 Tage ab Versuchbeginn, der Versuch erstreckte sich insgesamt über 40 Tage. Bei jeder Testperson wurde immer dieselbe Stelle (wasserfest auf der Haut markiert) ausgewertet. Jede Testperson erhielt sowohl die D₂O Creme wie auch die H₂O Creme an räumlich hinreichend getrennten und wasserfest markierten Bereichen des oberen Rückenbereichs aufgetragen, welche die zur Zählung verwendeten Bereiche umfassten.

### Figur 4:

Mittlere Anzahl von Komedonen in einen quadratischen, 10 x10 cm großen Hautgebiet auf dem oberen Rückenbereich von 14 Testpersonen mit mittelschwerer Akne vulgaris als Funktion der Behandlungszeit (Versuchstage) ) mit einer D₂O Creme (runde Symbole) und mit einer H₂O Creme (quadratische Symbole). Die Zählung der nicht-entzündeten Follikel erfolgte alle 5 Tage ab Versuchbeginn, der Versuch erstreckte sich insgesamt über 40 Tage Bei jeder Testperson wurde immer dieselbe Stelle (wasserfest auf der Haut markiert) ausgewertet. Jede Testperson erhielt sowohl die D₂O Creme wie auch die H₂O Creme an räumlich hinreichend getrennten und wasserfest markierten Bereichen des oberen Rückenbereichs aufgetragen, welche die zur Zählung verwendeten Bereiche umfassten.

### Figur 5:

Mittlere Anzahl von Papeln und Papulopusteln in einen quadratischen, 10 x10 cm großen Hautgebiet auf dem oberen Rückenbereich von 10 Testpersonen mit mittelschwerer Akne vulgaris als Funktion der Behandlungszeit (Versuchstage) mit einer D₂O Agarose Hydrogelfolie (runde Symbole) mit einer H₂O Agarose Hydrogelfolie (quadratische Symbole) welche direkt auf der Haut aufgebracht und alle 5 Tage gewechselt wurde. Die Zählung der entzündeten Follikel erfolgte alle 5 Tage ab Versuchbeginn, der Versuch erstreckte sich insgesamt über 40 Tage Bei jeder Testperson wurde immer dieselbe Stelle (wasserfest auf der Haut markiert) ausgewertet Jede Testperson erhielt sowohl die D₂O Agarose Hydrogelfolie wie auch die D₂O Agarose Hydrogelfolie an räumlich hinreichend getrennten und wasserfest markierten Bereichen des oberen Rückenbereichs aufgebracht, welche mit den zur Zählung verwendeten Bereichen identisch waren.

### Figur 6:

Mittlere Anzahl von Komedonen in einen quadratischen, 10 x10 cm großen Hautgebiet auf dem oberen Rückenbereich von 10 Testpersonen mit mittelschwerer Akne Vulgaris als Funktion der Behandlungszeit (Versuchstage) mit einer D₂O Agarose Hydrogelfolie (runde Symbole) mit einer H₂O Agarose Hydrogelfolie (quadratische Symbole) welche direkt auf der Haut aufgebracht und alle 5 Tage gewechselt wurde Die Zählung der nicht-entzündeten Follikel erfolgte alle 5 Tage ab Versuchbeginn, der Versuch erstreckte sich insgesamt über 40 Tage. Bei jeder Testperson wurde immer dieselbe Stelle (wasserfest auf der Haut markiert) ausgewertet Jede Testperson erhielt sowohl die D₂O Agarose Hydrogelfolie wie auch die D₂O Agarose Hydrogelfolie an räumlich hinreichend getrennten und wasserfest markierten Bereichen des oberen Rückenbereichs aufgebracht, welche mit den zur Zählung verwendeten Bereichen identisch waren

### Beispiele

### Beispiel 1: Herstellung von wasserhaltigen Gelen

Die Herstellung von wasserhaltigen Gelen erfolgte unter sterilen Bedingungen. Es wurde 3,0 Gew -% Agarose m reinem D₂O, in reinem H₂O oder in einer Mischung aus D₂O und H₂O gelost und die Lösung anschließend auf 90°C erhitzt Das verwendete D₂O der Firma Sigma-Aldrich (München) wies eine Isotopenreinheit von 98,5% auf Die heiße Lösung wurde in Petrischalen auf 3,0 mm Höhe gegossen und abgekühlt. Die so erhaltenen D₂O-Agarosegele, H₂O-Agarosegele oder D₂O/H₂O-Agarosegele wurden unter sterilen Bedingungen bei 4 °C gelagert Alternativ wurden unter Sterilbedingungen Acrylanudgele (5% Acrylamid) hergestellt, wobei reines D₂O, reines H₂O oder eine Mischung aus D₂O und H₂O vor Zugabe des Acrylamids (mit 2,4% bis-Acrylamid) entgast und auf 40 °C erwärmt wurde Nach Zugabe von Acrylamid und bis-Acrylamid wurde die Lösung gemischt (Vortex Mischer, 1 min, bei 200 U/min) und anschließend die Katalysatoren Tetramethyl-ethylendiamin (TEMES; 1,0%) und Ammoniumpersulfat (AP , 0,1%) zugegeben, gefolgt von 10 Sek Mischen Dann wurden die Gele in Petrischalen gegossen (Höhe des Gels 3 mm) und eine Schicht Butanol vor dem Aushärten darüber gelagert, um eine besonders glatte Geloberfläche zu erreichen. Zum Aushärten wurden die Gele für 2 Stunden bei 40° C gelagert

Für die jeweiligen Gele (Agarose und Acrylamid) wurde bei Bedarf zu dem reinen D₂O, reinen H₂O bzw der Mischung aus D₂O und H₂O vor Zugabe des Gelbildners 15 Vol-% Propylenglycol zugefügt, um mikrobielle Verunreinigungen bei der Gelapplikation zu unterdrücken.

### Beispiel 2: Herstellung eines okklusiven Depot-Pflasters mit D₂O oder H₂O als aktivem Wirkstoff

Kommerziell erhältliche Tegaderm Pflaster der Firma 3M (Neuss, Deutschland) in der Größe 6 x 7 cm wurden für die Präparation verwendet Von einem ersten Pflaster wurde der Papprahmen auf der dystalen Seite entfernt und m 6 getrennten Ansätzen ein nach Beispiel 1 hergestelltes Agarosegel oder Acrylamidgel (Durchmesser 2,5 cm, Dicke 3 mm) auf D₂O- oder H₂O- Basis wurde anschließend auf diese Seite zentrisch aufgebracht.

Die 6 Ansätze waren die folgenden:
Ansatz 1. Agarosegel + D₂O
Ansatz 2 Agarosegel + H₂O
Ansatz 3 Agarosegels + 1:1 Mischung aus D₂O und H₂O
Ansatz 4: Acrylamidgel + D₂O
Ansatz 5 Acrylamidgel + H₂O
Ansatz 6 Acrlyamidgel + 1:1 Mischung aus D₂O und H₂O

Auf das Gel wurde dann von oben eine kreisförmig geschnittene Schicht Parafilm (Durchmesser 3 cm) zentrrsch aufgelegt und ein zweites Tegaderm Pflaster, bei dem zuvor die Schutzfolie auf der Klebstoffseite (proximale Seite) entfernt worden war, wurde mit der Kleberseite zum Gel/Parafilm Schichtsystem orientiert auf der dystalen Seite des ersten Pflasters deckungsgleich aufgebracht und befestigt. Die fertigen Depotpflaster wurden bis zu ihrem Einsatz bei 4°C gelagert. Sämtliche Präparationen wurden unter sterilen Bedingungen durchgeführt.

### Beispiel 3: Erzeugung von D₂O-Aerosolen

Für die Erzeugung von Aerosolen wurden ausschließlich Geräte verwendet, die Stand der Technik sind Verwendet wurde ein Pari LC Plus Universal Vemebler (PARI GmbH, 82319 Starnberg, Deutschland) in Kombination mit einem Pari Universal Kompressor der 200 mg/min polydisperses Aerosol mit einer mittleren Partikelgröße (medianer Massendurchmesser) von 2,5 µm für reines H₂O und D₂O und von 2,5 - 4,5 µm für H₂O bzw. D₂O erzeugte (Betriebsdruck 2,0 bar, Durchflussmenge der Kompressorluft war 6,0 l/min.) Die Partikelgrößenmessung erfolgte mittels dynamischer Lichtstreuung in einer Durchfluß-Küvette Die Aerosolerzeugung erfolgte bei einer Temperatur von 37°C durch entsprechende Thermostatierung des Verneblers in einem Wasserbadthermostaten.

### Beispiel 4: Herstellung einer wasserhaltigen D₂O-Creme

Eine kommerziell verfügbare kolloide Basis zur Cremeherstellung (Avicel CL611 von FHG Pharmaceuticals, Philadelphia, USA) wurde entweder mit D₂O, mit H₂O oder mit einer Mischung aus D₂O und H₂O (jeweils gepuffert mit 50 mM Phosphat Puffer bei pH 7,0) vermischt. Hierzu wurden 2,0 Gew-% Avicel CL611 mittels eines Labormixers (Lighnin-Mixer von Aldrich Chemical Co., Milwaukee, USA) bei 500 U/min. in das auf 70 °C vorgewärmte reine D₂O, reine H₂O bzw. Mischung aus D₂O und H₂O gegeben und für 2 min. gemischt. Anschließend wurde die Drehzahl auf 1000 U/min erhöht und für weitere 10 min gemischt. Es wurden ebenfalls mehrere Cremes hergestellt, die zusätzlich weitere Bestandteile, insbesondere pharmazeutische Wirkstoffen (wie Kortikoide) enthielten. Hierzu wurden diese weiteren Bestandteile anschließend nach dem Mischen der o.g. jeweiligen Komponenten in die Creme-Basis gegeben und noch einmal 10 min. bei 1000 U/min gemischt. Das Ergebnis war eine homogene, langzeitstabile Creme.

### Beispiel 5: Einfluß von D₂O und H₂O auf die Proliferationsrate von humanen Hautzellinien in Kultur

Der Einfluß von D₂O und H₂O auf die Proliferationsrate von Karzinomzellen und normalen Zellen wurde an Zellkulturen überprüft. Hierzu wurden humane TE 354.T Epithelzelllinen, abgeleitet von dem humanen Basalzellkarzinom (American Type Culture Collection (ATCC), Rockville, USA), verwendet. Als Kontrolle wurden normale humane Epithelzellinien von der Haut desselben Individuums (TE 353.sk von ATCC) verwendet. Die Zellen wurden in 96- Well-Zellkutturplatten überführt (10⁴ Zellen pro Well) und bis Subconfluenz wachsen gelassen. Das hierfür verwendete Medium war Dulbecco's modifiziertes Eagles Medium (DMEM), versetzt mit 10% Wärme-deaktivierten, fötalen Kälberserum (LGC Promochem, Wesel, Deutschland). Es erfolgte ein Medienwechsel, erstmals 2 Tage nach Einbringung der Zellen und anschließend alle 48 Stunden. Im subconfluenten Stadium der Zellkultur beider Zelllinien wurde dem Medium 30% D₂O, bezogen auf das Gesamtmedium, zugesetzt. Bei einer Kontrollgruppe beider Zelllinien wurde anstelle von D₂O die identische Menge H₂O zugegeben. Anschließend wurde für beide Zelllinien mittels lichtmikroskopischer Zellzählung (Zellzahl/Fläche) unter Verwendung eines Haemozytometers eine Nullpunktsmessung als Basiswert für die Bestimmung der Zellverdopplungsrate durchgeführt 48 Stunden nach der Zugabe von D₂O bzw. H₂O wurden jeweils 6 Wells von Probe und Kontrolle vergleichend lichtmikroskopisch bezüglich Zellvermehrung untersucht. Danach wurden alle 24 Stunden für die Dauer einer Woche diese Untersuchungen fortgesetzt. Aus diesen Werten wurde die mittlere Zellverdopplungsrate n aus dem Anstieg einer graphischen Auftragung von log{Zellzahl} vs. Zeit entsprechend der Gleichung n = 3 32 (log Nₜ - log N₀) bestimmt, Hierbei ist Nₜ die nach der Zeit t gemessene Zellzahl und No die zum Zeitpunkt der Zugabe von D₂O bzw H₂O (Nullpunktsmessung) bestimmte Zellzahl. Aus der Mittelung der Ergebnisse über jeweils 6 Wells pro Zeitpunkt wurde die gemittelte Zellverdopplungsrate <n> bestimmt. Die Ergebnisse sind m **Tabelle 1** zusammengefasst. Eine überproportionale Absenkung von <n> für die Karzinomzellen im Vergleich zu normalen Zellen durch den Zusatz von D₂O ist evident.

**Tabelle 1:**

| Karzinomzellen | | Normale Zellen | |
|---|---|---|---|
| <n> D₂O | <n> H₂O | <n> D₂O | <n> H₂O |
| 0,23 ± 0,02 | 0,92 ± 0,08 | 0,41 ± 0,04 | 0,65 ± 0,04 |

| | | | |
|---|---|---|---|
| Einfluß von D₂O und H₂O auf die mittlere Zellverdopplungsrate <n>D₂O bzw <n>H₂O von Zellkulturen humaner TE 354 T Epithelzelllinen, abgleitet von dem humanen Basalzellkarzinom (Karzinomzellen) und von normalen Epithelzellinien der Haut (Normale Zellen) desselben Individuums (TE 353.sk) | | | |

### Beispiel 6: Einfluß von D₂O und H₂O auf die Entwicklung von Narben der Haut

Acrylamidgele mit reinem D₂O und reinem H₂O (Kontrolle) wurden entsprechend Beispiel 1 präpariert, wobei das D₂O bzw. H₂O 15 Vol-% Propylenglycol enthielt. Die Dicke der Gele betrug 2 mm, es wurden aus dem Gel 2 x 4 cm große Stücke zugeschnitten.

9 Kaninchen (Neuseeland, weiß) wurden am Ohr, jeweils auf der ventrikulären Seite, durch Einschnitte von 6 mm Durchmesser verletzt und die entstandenen Wunden wurden ab dem 16 Tag nach der Verwundung bei 6 Kaninchen mit den obigen Gelen behandelt, und zwar bei 3 Kaninchen mit D₂O-Acrylamidgelen, bei 3 Kaninchen mit H₂O-Acrylamidgelen, und bei 3 Kaninchen erfolgte die Heilung unbehandelt Von den 6 Kaninchen wurden 3 mit einem D₂O-Acrylamidgel und 3 mit einem H₂O-Acrylamidgel behandelt. Hierzu wurde das entsprechende Gel auf der Wunde aufgebracht und mit einer 1 mm dicken selbstklebenden Silikonfolie (Biodermis) fixiert. Der Wechsel der Gele erfolgte alle 2 Tage Am 24. Tag nach der Verwundung erfolgte bei allen 9 Kaninchen eine 3D-Narbenvermessung mittels Streifenprojektion (System ATOS-1 der Fa. GOM, Bibertal bei Ulm, Deutschland) bei der Narbenfläche und hypertrophes Narbenvolumen berührungslos bestimmt wurden. Die Ergebnisse sind in **Tabelle 2** zusammengestellt. Es erwies sich eine signifikant geringere Ausbildung der Narbe bzgl. Narbenvolumen für die drei mit D₂O-Acrylamidgel behandelten Kaninchen.

**Tabelle 2:**

| | Narbenfläche (mm²) | Narbenvolumen (mm³) |
|---|---|---|
| D₂O-Gel | 58± 7 | 83±14 |
| H₂O-Gel | 72±8 | 124±16 |
| Kontrolle | 78±8 | 158±17 |

| | | |
|---|---|---|
| Narbenfläche und hypertrophes Narbenvolumen am 24 Tag nach der Verletzung für auf die Wunde am 16 Tag nach der Verletzung aufgebrachte Acrylamidgele auf D₂O Basis (D₂O-Gel) und auf H₂O Basis (H₂O-Gel) sowie für Narben, die nach der Verletzung nicht weiter behandelt wurden (Kontrolle) Die angegebenen Werte sind Mittelwerte über jeweils 6 Narben und die für den Mittelwert errechnete Standardabweichung. | | |

### Beispiel 7: Einfluß von D₂O- und H₂O- Depot-Pflastern auf Psoriasis

Die in Beispiel 2 beschriebenen, okklusiven Depot-Pflaster wurden auf 2-3 cm Durchmesser große Psoriasisgebiete der humanen Haut (Arme und Beine) von Probanden aufgebracht und dort für 3 Tage belassen. Insgesamt wurden bei denselben Individuen jeweils 5 Hautstellen mit D₂O- und weitere S mit H₂O-Pflastern bedeckt. Es wurde für jeweils 3 der D₂O- bzw. H₂O-Pflaster eine Zubereitung auf Basis eines Agarosegels gem Beispiel 1 und für jeweils 3 der D₂O-bzw H₂O-Pfläster eine Zubereitung auf Basis eines Acrylamidgels gem. Beispiel 1 verwendet Die ausgewählten Gebiete zeichneten sich durch intensive(n) Juckreiz, Hautrötung und Schuppigkeit aus Nach Entfernen der Pflaster, 72 Stunden nach ihrer Applikation, wurden die behandelten Hautgebiete dermaskopisch analysiert Bei den mit D₂O-Pflastern behandelten Gebieten war die behandelte Haut visuell nicht mehr von der umgebenden, gesunden Haut unterscheidbar, Juckreiz, Rötung und Schuppigkeit waren vollständig verschwunden. Bis zu einem Beobachtungszeitraum von vier Wochen nach der Entfernung der Pflaster konnte keine Rückkehr der Symptome beobachtet werden Für die H₂O-Pflaster wurde ein leichter Rückgang von Juckreiz, Rötung und ein Verschwinden der Schuppigkeit festgestellt, allerdings kehrten alle drei Symptome 2 Tage nach der Entfernung der Pflaster wieder zurück und waren nach weiteren 2 Tagen vollständig wie vor der Behandlung manifest, d.h. in gleichem Grad vorhanden wie vor der Behandlung. Die Ergebnisse erwiesen sich als unabhängig von der Art des für die Gele verwendeten Polymers, d h. es konnten keine Unterschiede für Agarose-order Acrylamidgele beobachtet werden.

### Beispiel 8: Einfluß von D₂O- und H₂O -haltigen Cremezubereitungen auf Psoriasis

Nach Beispiel 4 zubereitete Cremes mit entweder reinem D₂O oder reinem H₂O wurden alle 6 Stunden auf 2-3 cm große Psoriasis-Hautareale humaner Haut von Probanden dünn aufgetragen. Die pro Auftragung aufgebrachte Menge an Creme entsprach einer D₂O- bzw H₂O-Menge von 60-70 µl pro Quadratzentimeter Haut Die Auftragung wurde über 3 Tage fortgesetzt und nach 72 Stunden beendet. Anschließend wurden die behandelten Hautareale dermaskopisch analysiert. Für die D₂O-haltige Creme war die behandelte Haut nach 3 Tagen visuell nicht von der umgebenden, gesunden Haut unterscheidbar, Juckreiz, Rötung und Schuppigkeit waren weitgehend verschwunden. Diese Winkung hielt für den Beobachtungszeitraum von 4 Wochen an Für die H₂O-haltige Creme wurde ein leichter Rückgang der Rötung und ein(e) leicht verringerte(r) Schuppigkeit und Juckreiz festgestellt, aber mit einer Rückkehr der Symptome nach 3 Tagen

### Beispiel 9: Herstellung von Hydrogelen auf Basis von Acrylsäure

Es wurde 1,0 Gew -% Carbopol 980 (Hersteller: Noveon, In., 9911 Brecksville Rd , Cleveland, OH 44141-3247, USA) und 0,1 Gew-% Sorbinsäure in getrennten Ansätzen in reinem D₂O, in reinem H₂O oder in einer Mischung aus D₂O und H₂O (1:1) durch Rühren gelost und anschließen durch Pipettieren von 10 M NaOH Lösung auf einen pH Wert von 6,8 titriert Anschließend wurden die durch die NaOH Zugabe infolge Quervernetzung der Polyacrylsäure über ihre Carboxylgruppen mit den alkalischen Hydroxyl-Gruppen entstandenen farblosen, transparenten und optisch klaren Acrylsäuregele (Carbopolgele) (D₂O-Carbopolget, H₂O-Carbopolgel, D₂O/H₂O-Carbopolgel) bei Raumtemperatur bis zur weiteren Verwerdung, für mindestens 24 Stunden, gelagert. Das in diesem und allen folgenden Beispielen verwendete D₂O der Firma Sigma-Aldrich (München) wies eine Isotopenreinheit von 99,0% auf.

### Beispiel 10: Herstellung von Hydrogelen auf der Basis von Siloxanen (Silicon)

Es wurde 3,0 Gew-% Hexamethyldisiloxane (Handelsname SILMOGEN CARRIER von DOW Corning) und 1 Gew-% Ethanol in getrennten Ansätzen in reinem D₂O, in reinen H₂O oder in einer Mischung aus D₂O und H₂O (1:1) durch Rühren gelöst. Diese Lösungen wurden anschließend sofort im Gewichtsverhältnis 1 2 (Siliconlösung-Carbopolgel) unter kräftigen Rühren mit dem nach Beispiel 1 hergestellten Gel (Carbopolgel) gemischt, bis optisch transparente Gele (Silicongele) (D₂O-Silicongel, H₂O-Silicongel, D₂O/H₂O-Silicongel) entstanden waren Die Lagerung der Gele erfolgte bei Raumtemperatur bis zur weiteren Verwendung, für mindestens 24 Stunden.

### Beispiel 11: Herstellung von Hydrogel auf der Basis von Alginaten

Es wurde 2,0 Gew-% Alginsäurenatriumsalz (Na-Alginat) (Hersteller: Röhm GmbH Darmstadt, Deutschland) und 0,1 Gew-% Sorbinsäure in getrennten Ansätzen in reinem D₂O, in reinem H₂O oder m einer Mischung aus D₂O und H₂O durch Rühren dispergiert und anschließend durch Pipettieren von 10 M NaOH Lösung auf einen pH Wert von 7,0 titriert. Die entstandenen gelblich-braunen, transparenten Gele (Alginatgele) (D₂O-Alginatgel, H₂O-Alginatgel, D₂O/H₂O-Alginatgel) wurden bei Raumtemperatur bis zur weiteren Verwendung, für mindestens 24 Stunden, gelagert

### Beispiel 12:Herstellung von Hydrogel auf der Basis von PVA

Es wurde 20 Gew-% Polyvinylalkohol (PVA C-25, Shin-Etsu Chemical Co, Japan) in getrennten Ansätzen in reinem D₂O, in reinem H₂O oder in einer (1 :1) Mischung aus D₂O und H₂O durch Rühren gelöst. Anschließend wurden die Lösungen 5 Gefrier-Auftau-Zyklen ("freeze-thaw-cycles") unterworfen. Das Ergebnis waren Gele (PVA-Gele) (D₂O-PVA-Gel, H₂O-PVA-Gel, D₂O/H₂O-PVA-Gel) mit gummiartigen Eigenschaften, welches in 2 mm dünne Scheiben geschnitten wurde Die Gele wurden bei Raumtemperatur bis zur weiteren Verwendung, für mindestens 24 Stunden, gelagert

### Beispiel 13: Herstellung von Hydrogel - Folien bzw. Platten auf der Basis von Agarose

Es wurde 3,0 Gew.-% Agarose , versetzt mit 0,1 Gew-% Sorbinsäure, in getrennten Ansätzen in reinem D₂O, in reinem H₂O oder in einer (1:1) Mischung aus D₂O und H₂O gelöst und die Lösungen anschließend auf 90°C erhitzt. Das verwendete D₂O der Firma Sigma-Aldrich (München, Deutschland) wies eine Isotopenreinheit von 98,5% auf Die heißen Lösungen wurde in geeignete Petrischalen auf 1,0 - 1,5 mm Höhe gegossen und abgekühlt. Die so erhaltenen Gele (Agarosegele) (D₂O-Agarosegele, H₂O-Agarosegele, D₂O/H₂O-Agarosegele) wurden unter sterilen Bedingungen bei 4 °C gelagert.

### Beispiel 14: Herstellung von Hydrogel - Folien bzw. Platten auf der Basis von Acrylamid

Es wurden Acrylamidgele (5% Acrylamid) hergestellt, wobei in getrennten Ansätzen reines D₂O, reines H₂O oder eine (1:1) Mischung aus D₂O und H₂O vor Zugabe des Acrylamids (das 2,4% bis-Acrylamid enthielt) entgast und auf 40°C erwärmt wurde. Nach Zugabe von Acrylamid und bis-Acrylamid wurden die Lösungen gemischt (Vortex Mischer, 1 min. bei 200 U/min) und anschließend die Katalysatoren Tetramethyl-ethylendiamin (TEMES; 1,0%) und Ammoniumpersulfat (AP; 0,1%) zugegeben, gefolgt von 10 Sek. Mischen. Dann wurden die Gele in Petrischalen gegossen (Höhe des Gels 1,0-1,5 mm) und für 2 Stunden bei 40°C gelagert. Anschließend wurden die Gele (D₂O-Acrylamidgel, H₂O-Acrylamidgel, D₂O/H₂O-Acrylamidgel) gewaschen, wobei zum Waschen das analoge Wassergemisch wie zum Hydratisieren des Gels (reines D₂O, reines H₂O oder eine Mischung aus D₂O und H₂O) verwendet wurde. Die Gele wurden bei Raumtemperatur bis zur weiteren Verwendung, für mindestens 24 Stunden, gelagert.

### Beispiel 15: Herstellung einer D₂O-haltigen Creme

Zu 50 Gramm Asche® Basiscreme (Hersteller: Asche Chiesi GmbH, Hamburg, Deutschland) wurde bei 40°C unter ständigen Rühren langsam D₂O hinzugegeben, bis ein Gewichtsanteil vom 45% D₂O (bezogen auf das Ausgangsgewicht der Creme) in der homogenen Mischung erreicht war. Die Creme wurde anschließend auf Raumtemperatur abgekühlt und luftdicht verschlossen gelagert.

### Beispiel 16: Wirksamkeit von D₂O Hydrogel zur Therapie von Akne vulgaris im klinischen Versuch, Vergleich mit H₂O Placebo

Es wurden 32 gesunde Freiwillige im Alter von 15-24 Jahren (16 weiblich, 16 männlich) für diese Studie ausgewählt. Alle ausgewählten Testpersonen hatten eine Historie von Akne vulgaris im Rückenbereich. Alle Testpersonen wiesen im Rückenbereich mittelschwere Akne vulgaris sowohl mit entzündlichen (Papeln und Papulopusteln) als auch mit nicht-entzündlichen (Kornedonen) Bereichen auf. Keine der Testpersonen benutzte eine Woche vor oder während des auf 40 Tage begrenzten Tests antibakterielle Therapeutika oder immunmodulierende Therapeutika.

Allen Testpersonen wurden 2 Gele zur Verfügung gestellt: Es handelte sich dabei um die nach Beispiel 9 hergestellten Gele auf Acrylsäure-Basis, wobei das Probengel mit D₂O und das Placebogel mit H₂O hergestellt wurde. Zur Vermeidung von Verwechslungen war das Placebogel mit einem Lebensmittelfarbstoff markiert worden. Die Testpersonen wurden angewiesen, beide Gele morgens und abends auf dem Rücken um eine jeweils vorbezeichnete Stelle (wasserfeste Markierung auf der Haut) im Radius von mindestens 10 cm dünn aufzutragen bzw auftragen zu lassen, das Probengel um eine Markierung auf der linken Rückenseite und das Placebogel um eine Markierung auf der rechten Rückenseite. Diese markierten Stellen (je eine auf der- linken und eine auf der rechten Rückenhälfte) wurden am Beginn des klinischen Versuchs wegen ihrer besonders hohen Dichte an Papeln und Papulopusteln ausgewählt. Die Testpersonen wurden alle 5 Tage dermatologisch begutachtet. Hierbei wurde die Zahl der Papeln und Papulopusteln sowie die Zahl der Komedonen auf einer quadratischen, 10 x 10 cm großen Fläche um das Zentrum der wasserfest markierten Stellen auf der linken und rechten Rückenhälfte bestimmt. Diese Bestimmung erfolgte durch Aufsetzen eines entsprechend großen Rahmens aus Plexiglas, bei jeder Testperson und jeder Begutachtung immer auf derselben Stelle (nicht-abwaschbare, Markierung auf der Haut in Übereinstimung mit Markierung auf dem Plexiglas)). Am Rand des Rahmens liegende Objekte wurden mitgezählt, falls sie mit mindestens 50% ihrer Fläche im Auswertebereich lagen.

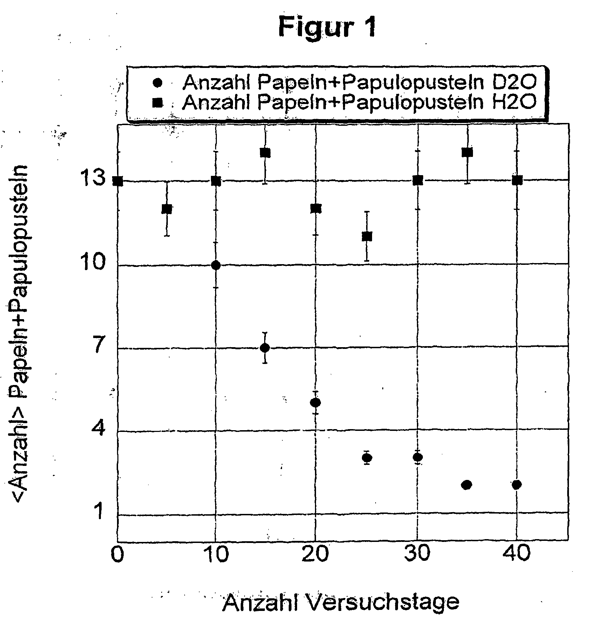

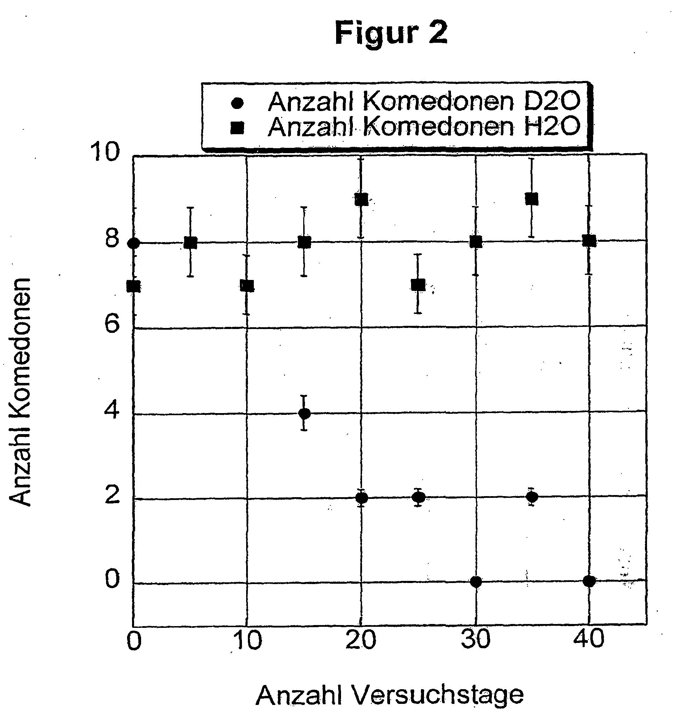

Die Ergebnisse sind in Figur 1 für Papeln bzw. Papulopusteln und in Figur 2 für Komedonen graphisch dargestellt.

Zusammenfassend kann man aus den Ergebnissen eine deutliche und nachhaltige Wirkung von D₂O Gel zur Unterdrückung von entzündlichen und nichtentzündlichen Bereichen der Akne vulgaris im Vergleich zum Placebo (H₂O) schlussfolgern. Während die unterschiedliche Wirkung in den ersten 10 Tagen nach Therapiebeginn nur gering ist, wird sie ab dem 15. Therapietag deutlicher und ab dem 20 Therapietag sehr deutlich. Insbesondere zeigt sich, dass entzündliche Bereiche im Endstadium (aufgeplatztes Follikel) nur wenig beeinflusst werden, die Bildung neuer Papeln bzw Papulopusteln wie auch neuer Komedone hingegen signifikant verringert wird.

### Beispiel 17: Wirksamkeit von D₂O Creme zur Therapie von Akne Vulgaris im klinischen Versuch, Vergleich mit H₂O Placebo

Es wurden 14 gesunde Freiwillige im Alter von 16-24 Jahren (6 weiblich, 8 männlich) für diese Studie ausgewählt. Alle ausgewählten Testpersonen hatten eine Historie von Akne vulgaris im Rückenbereich. Alle Testpersonen wiesen im Rückenbereich mittelschwere Akne vulgaris sowohl mit entzündlichen (Papeln und Papulopusteln) als auch mit nicht-entzündlichen (Komedonen) Bereichen auf. Keine der Testpersonen benutzte eine Woche vor oder während des auf 40 Tage begrenzten Tests antibakterielle Therapeutika oder immunmodulierende Therapeutika.

Allen Testpersonen wurden 2 Cremes zur Verfügung gestellt. Es handelte sich dabei um die nach Beispiel 15 hergestellten Creme auf Basis einer Asche Basiscreme, wobei die Probencreme mit D₂O und die Placebocreme mit H₂O hergestellt wurde. Zur Vermeidung von Verwechslungen war die Placebocreme mit emem Lebensmittelfarbstoff markiert worden Die Testpersonen wurden angewiesen, die Creme morgens und abends auf dem Rücken um eine jeweils vorbezeichnete Stelle (wasserfeste Markierung auf der Haut) im Radius von mindestens 10 cm dunn aufzutragen bzw. auftragen zu lassen, die Probencreme um eine Markierung auf der linken Rückenseite und die Placebocreme um eine Markierung auf der rechten Rückenseite. Diese markierten Stellen (je eine auf der linken und eine auf der rechten Rückenhälfte) wurden am Beginn des klinischen Versuchs wegen ihrer besonders hohen Dichte an Papeln und Papulopusteln ausgewählt. Die Testpersonen wurden alle 5 Tage dermatologisch begutachtet. Hierbei wurde die Zahl der Papeln und Papulopusteln sowie die Zahl der Komedonen auf einer quadratischen, 10 x 10 cm großen Fläche um das Zentrum der wasserfest markierten Stellen auf der linken und rechten Rückenhälfte bestimmt. Diese Bestimmung erfolgte durch Aussetzen eines entsprechend großen Rahmens aus Plexiglas, bei jeder Testperson und jeder Begutachtung immer auf derselben Stelle (nicht-abwaschbare Markierung auf der Haut in Ubereinstimung mit Markierung auf dem Plexiglas)) Am Rand des Rahmens liegende Objekte wurden mitgezählt, falls sie mit mindestens 50% ihrer Fläche im Auswertebereich lagen.

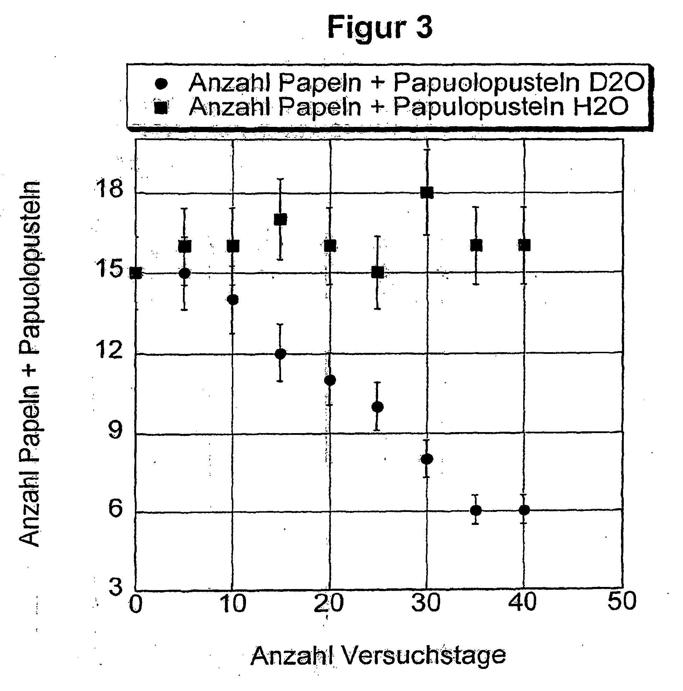

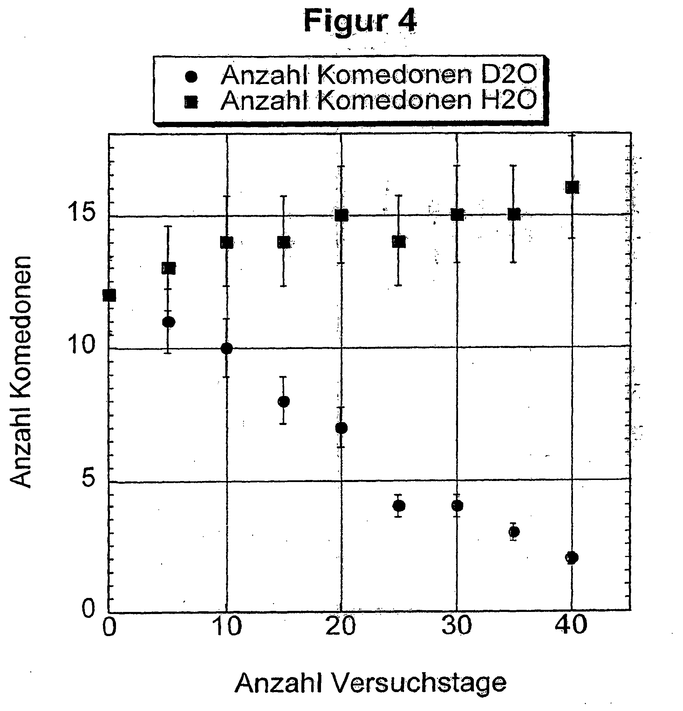

Die Ergebnisse sind in Figur 3 für Papeln bzw. Papulopusteln und in Figur 4 für Komedonen graphisch dargestellt.

### Beispiel 18: Wirksamkeit von D₂O Hydregelfolien zur Therapie von Akne Vulgaris im klinischen Versuch, Vergleich mit H₂O Placebo

Es wurden 10 gesunde Freiwillige im Alter von 15-24 Jahren (6 weiblich, 4 männlich) für diese Studie ausgewählt. Alle ausgewählten Testpersonen hatten eine Historie von Akne vulgaris im Ruckenbereich. Alle Testpersonen wiesen im Rückenbereich mittelschwere Akne vulgaris sowohl mit entzündlichen Papeln und Papulopusteln) als auch mit nicht-entzündlichen (Komedonen) Bereichen auf. Keine der Testpersonen benutzte eine Woche vor oder während des auf 40 Tage begrenzten Tests antibakterielle Therapeutika oder immunmodulierende Therapeutika

Nach Beispiel 13 hergestellte Agarose Hydrogelfolien der Dicke 2 mm, entweder aus D₂O-(Probengel) oder H₂O- (Placebogel) Basis wurden in 10 x 10 cm große Stücke geschnitten und den Testpersonen auf zuvor markierte Stellen (wasserfeste Markierung) auf dem Rücken , zentrisch um die markierte Stelle, aufgelegt und mit Tegaderm Pflaster fixiert. Diese markierten Steilen (je eine auf der linken und eine auf der rechten Rückenhälfte) wurden am Beginn des klinischen Versuchs wegen ihrer besonders hohen Dichte an Papeln und Papulopusteln ausgewählt. Auf der linken Rückenhälfte wurde das Probengel und auf der rechten Rückenhälfte das Placebogel, aufgebracht. Die Testpersonen wurden alle 5 Tage dermatologisch begutachtet und anschließend das Agarose-Gel gegen ein jeweils neues Gel der gleichen Größe ausgetauscht. Hierbei wurde die Zahl der Papeln und Papulopusteln sowie die Zahl der Komedonen auf einer quadratischen, 10 x 10 em großen Fläche um das Zentrum der (wasserfest markierten Stellen auf der linken und rechten Rückenhälfte bestimmt. Diese Bestimmung erfolgte durch Aufsetzen eines entsprechend großen Rahmens aus Plexiglas (nach Entfernung des Agarose Gels), bei jeder Testperson und jeder Begutachtung immer auf derselben Stelle (nicht-abwaschbare Markierung auf der Haut in Übereinstimung mit Markierung auf dem Plexiglas)). Am Rand des Rahmens liegende Objekte wurden mitgezählt, falls sie mit mindestens 50% ihrer Fläche im Auswertebereich lagen.

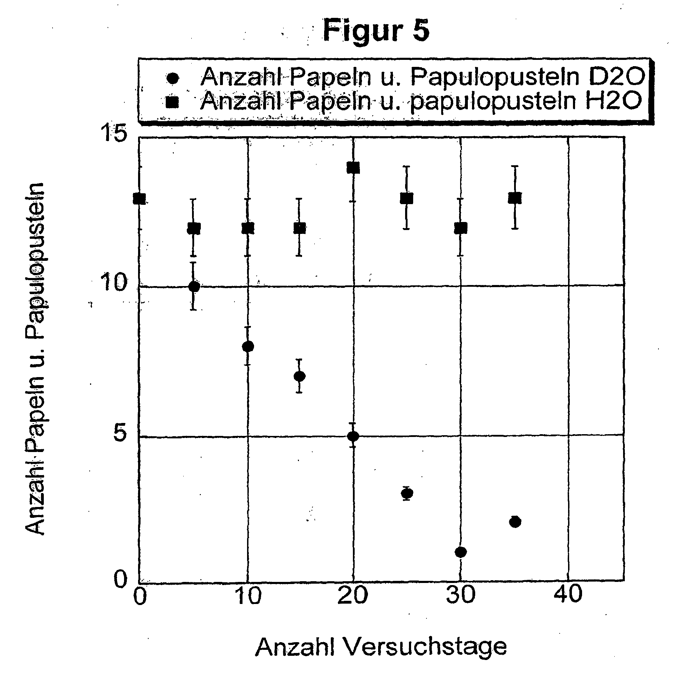

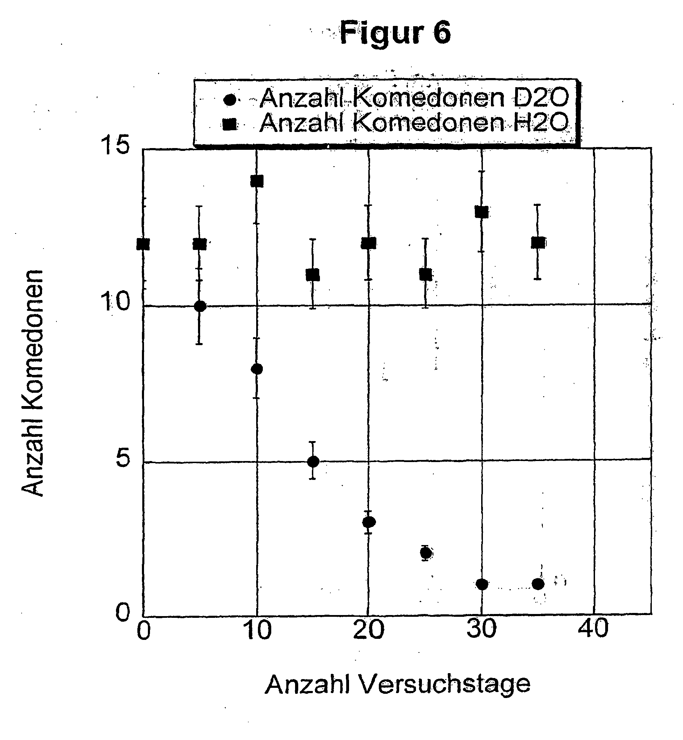

Die Ergebnisse sind in Figur 5 für Papeln bzw. Papulopusteln und in Figur 6 für Komedonen graphisch dargestellt.

### Beispiel 19: Wirksamkeit von D₂O Creme zur Therapie von diabetischen Fuß (neuropatisch-infizierter Fuß) im klinischen Versuch, Vergleich mit H₂O Placebo

Es wurden 12 Freiwillige im Alter von 25-62 Jahren (6 weiblich, 6 männlich) für diese Studie ausgewählt. Alle ausgewählten Testpersonen hatten eine Historie von Diabetes Mellitus (Typ 1 oder 2) und wiesen ausgeprägte Symptome des neuropatisch - infizierten diabetischen Fußes auf. Kennzeichnend für alle Testpersonen war eine massive Verhornung der Fußsolen.

Allen Testpersonen wurden 2 Cremes zur Verfügung gestellt: Es handelte sich dabei um die nach Beispiel 15 hergestellten Creme auf Basis einer Asche Basiscreme, wobei die Probencreme mit D₂O und die Placebocreme mit H₂O hergestellt wurde. Zur Vermeidung von Verwechslungen war die Placebocreme mit einem Lebensmittelfarbstoff markiert worden. Die Testpersonen wurden angewiesen, die Creme morgens und abends auf den am meisten betroffenen Fuß um zwei jeweils vorbezeichnete Stellen (wasserfeste Markierung auf der Hornhaut) im Radius von mindestens 2 cm dünn aufzutragen bzw auftragen zu lassen. Die Markierungen waren von unterschiedlicher Farbe und die Testpersonen waren angewiesen, die Probencreme um die rote Markierung und die Placebocreme um die schwarze Markierung aufzutragen. Die wasserfest markierten Stellen wurden vor Versuchsbeginn wegen ihrer besonders dicken Verhornung ausgewählt. Eine dermatologische Begutachtung erfolgte alle 10 Tage währen des insgesamt 40 tägigen klinischen Versuchs.

Die Ergebnisse können wie folgt zusammengefasst werden. Nach 10 Tagen ab Versuchsbeginn (zweite dermatologische Begutachtung) konnten keine signifikanten Unterschiede in der Verhornung um die beiden Markierungen beobachtet werden. Nach 20 Tagen ab Versuchsbeginn waren Anzeichen für eine langsamer nachwachsende Verhornung für die Probencreme gegenüber der Placebocreme zu sehen, die bei der vierten dermatologischen Begutachtung am Tag 30 nach Versuchbeginn deutlich sichtbar wurde, sowohl anhand der Hautelastizität wie auch ihrer Farbe. Am Tag 40 nach Versuchbeginn waren signifikante Unterschiede zwischen den beiden behandelten Bereichen sichtbar, die mit einer verringerten Dicke der Hornhautschicht der mit der Probencreme behandelten Bereiche im Vergleich zur Placebocreme erklärt werden können.

## Patentansprüche

1. D₂O zur Verwendung für die Prophylaxe und/oder Therapie von hyperproliferativen, nicht-malignen Erkrankungen der Haut, wobei die hyperproliferative, nicht-maligne Erkrankung der Haut ausgewählt ist aus Keratosen, insbesondere benigner lichenoider Keratose, palmoplantarer Keratose, follikulärer Keratose, Verruca seborrhoica und Lichen-planus-artiger Keratose, Porokeratose, wie Porokeratosis disseminata, Porokeratosis Mibelli, Porokeratosis naeviformis, Porokeratosis striata und Porokeratosis disseminata, aktinischer Keratose, epidermolytischer Hyperkeratose, Hyperkeratosis Lenticularis Perstans, Keratosis pilaris, ichthyose, Akne, wie Akne vulgaris, Akne inversa, Akne comedonica, Akne papula-pustulosa, Akne conglobata, Hidradentis suppurativea, Akne aestivalis, Akne cosmetica, Akne medicamentosa, Akne venenata und Akne tarda, und Hyperkeratose im Zusammenhang mit Diabetes mellitus, sowie Narben der Haut, insbesondere hypertrophen Narben und Keloiden.

2. D₂O zur Verwendung nach Anspruch 1 zur topischen Applikation auf die Haut.

3. D₂O zur Verwendung nach einem beliebigen der vorangehenden Ansprüche, wobei D₂O die Proliferation von Hautzellen, wie Keratinozyten, Epidermiszellen, Dermiszellen, Fibroblasten, Kollagenzellen, Bindegewebszellen und Melanozyten; hemmt und/oder inhibiert.

4. D₂O zur Verwendung nach einem beliebigen der vorangegangenen Ansprüche, wobei das D₂O in Kombination mit mindestens einem weiteren pharmazeutischen Wirkstoff und/oder mindestens einem weiteren nicht-pharmazeutischen Wirkstoff verwendet wird.

5. D₂O zur Verwendung nach einem beliebigen der vorangegangenen Ansprüche, zur topischen Applikation mit einem Pflaster oder einer Bandage.

6. D₂O zur Verwendung nach Anspruch 5, wobei das Pflaster oder die Bandage in Kombination mit mindestens einer Membran oder mindestens einer Folie verwendet wird, insbesondere mindestens einer mikro- oder nanoporösen Membran oder Folie.

7. D₂O zur Verwendung nach einem der Ansprüche 1 bis 5, wobei das D₂O als Aerosol oder als topische Formulierung vorliegt.

8. D₂O zur Verwendung nach Anspruch 7, wobei die Formulierung eine Salbe, eine Creme oder ein Gel ist.

9. Pflaster oder Bandage zur topischen Applikation, enthaltend D₂O und weiterhin mindestens einen weiteren pharmazeutischen Wirkstoff ausgewählt aus immunosuppressiven Wirkstoffen, wie Kortikoiden, und Wachstumsfaktoren.

10. Pflaster oder Bandage nach Anspruch 9 wobei das Pflaster ein Depot-Pflaster ist und wobei die Bandage eine Depot-Bandage ist.

11. Aerosol, welches eine Mischung aus D₂O und H₂O enthält zur topischen Applikation auf die Haut, wobei das Aerosol weiterhin mindestens einen weiteren pharmazeutischen Wirkstoff ausgewählt aus immunosuppressiven Wirkstoffen, wie Kortikoiden, und Wachstumsfaktoren enthält.

## Claims

1. D₂O for use for the prevention and/or treatment of hyperproliferative, non-malignant skin diseases, the hyperproliferative, non-malignant skin disease being selected from keratoses, in particular benign lichenoid keratosis, palmoplantar keratosis, follicular keratosis, seborrheic verruca and lichenplanus-like keratosis, porokeratosis, such as porokeratsis disseminata, porokeratosis of Mibelli, linear porokeratosis, porokeratosis striata and porokeratosis disseminata, actinic keratosis, epidermolytic hyperkeratosis, hyperkeratosis lenticularis perstans, keratosis pilaris, ichthyosis, acne, such as acne vulgaris, acne inversa, acne comedonica, acne papulopustulosa, acne conglobata, hidradenitis suppurativa, acne aestivalis, acne cosmetica, acne medicamentosa, acne venenata and acne tarda, and hyperkeratosis associated with diabetes mellitus, and scars of the skin, in particular hypertrophic scars and keloids.

2. D₂O for use according to claim 1 for topical administration onto the skin.

3. D₂O for use according to either one of the preceding claims, wherein D₂O restricts and/or inhibits proliferation of skin cells, such as keratinocytes, epidermal cells, dermal cells, fibroblasts, collagen cells, connective tissue cells and melanocytes.

4. D₂O for use according to any one of the preceding claims, wherein the D₂O is used in combination with at least one further pharmaceutical active ingredient and/or at least one further non-pharmaceutical active ingredient.

5. D₂O for use according to any one of the preceding claims for topical administration with a patch or a dressing.

6. D₂O for use according to claim 5, wherein the patch or dressing is used in combination with at least one membrane or at least one film, in particular at least one micro- or nanoporous membrane or film.

7. D₂O for use according to one of claims 1 to 5, wherein the D₂O is in the form of an aerosol or a topical formulation.

8. D₂O for use according to claim 7, wherein the formulation is an ointment, a cream or a gel.

9. A patch or dressing for topical administration, containing D₂O and furthermore at least one further pharmaceutical active ingredient selected from immunosuppressant active ingredients, such as corticoids, and growth factors.

10. A patch or dressing according to claim 9, wherein the patch is a time-release patch and wherein the dressing is a time-release dressing.

11. An aerosol which contains a mixture of D₂O and H₂O for topical administration onto the skin, wherein the aerosol furthermore contains at least one further pharmaceutical active ingredient selected from immunosuppressant active ingredients, such as corticoids, and growth factors.

## Revendications

1. D₂O pour l'utilisation pour la prophylaxie et/ou la thérapie de maladies hyperprolifératives non malignes de la peau, la maladie hyperproliférative non maligne de la peau étant choisie parmi les kératoses, en particulier, la kératose lichénoïde bénigne, la kératose palmoplantaire, la kératose folliculaire, les verrues séborrhéiques et la kératose de type lichen plan, la porokératose telle que la porokératose disséminée, la porokératose de Mibelli, la prokératose naeviforme, la porokératose striée et la porokératose disséminée, la kératose actinique, l'hyperkératose épidermolytique, l'hyperkératose lenticulaire persistante, la kératose pilaire, l'ichthyose, l'acné telle que l'acné vulgaire, l'acné inversée, l'acné comédonienne, l'acné papulopustuleuse, l'acné conglobata, l'hidradenitis suppurativa, l'acné estivale, l'acné cosmétique, l'acné médicamenteuse, l'acné venenata et l'acné tardive et l'hyperkératose associée au diabète sucré ainsi que les cicatrices cutanées, en particulier, les cicatrices hypertrophiques et les chéloïdes.

2. D₂O pour l'utilisation selon la revendication 1, pour l'application topique sur la peau.

3. D₂O pour l'utilisation selon l'une quelconque des revendications précédentes, le D₂O bloquant et/ou inhibant la prolifération de cellules cutanées telles que les kératinocytes, les cellules épidermiques, les cellules dermiques, les fibroblastes, les cellules de collagène, les cellules de tissu conjonctif et les mélanocytes.

4. D₂O pour l'utilisation selon l'une quelconque des revendications précédentes, le D₂O étant utilisé en combinaison avec au moins une autre substance active pharmaceutique et/ou au moins une autre substance active non pharmaceutique.

5. D₂O pour l'utilisation selon l'une quelconque des revendications précédentes, pour l'application topique avec un pansement ou un bandage.

6. D₂O pour l'utilisation selon la revendication 5, le pansement ou le bandage étant utilisé en combinaison avec au moins une membrane ou au moins une feuille, en particulier au moins une membrane ou feuille micro- ou nanoporeuse.

7. D₂O pour l'utilisation selon l'une des revendications 1 à 5, le D₂O se présentant sous forme d'aérosol ou en formulation topique.

8. D₂O pour l'utilisation selon la revendication 7, la formulation étant une pommade, une crème ou un gel.

9. Pansement ou bandage pour l'application topique, contenant du D₂O et en outre au moins une autre substance active pharmaceutique choisie parmi les substances actives immunosuppressives telles que les corticoïdes et les facteurs de croissance.

10. Pansement ou bandage selon la revendication 9, le pansement étant un pansement à libération retardée et le bandage étant un bandage à libération retardée.

11. Aérosol qui contient un mélange de D₂O et de H₂O pour l'application topique sur la peau, l'aérosol contenant en outre au moins une autre substance active pharmaceutique choisie parmi les substances actives immunosuppressives telles que les corticoïdes et les facteurs de croissance.
